(19) Europäisches Patentamt
European Patent Office
Office européen des brevets

(11) **EP 4 729 942 A2**

(12) **EUROPEAN PATENT APPLICATION**

(43) Date of publication:
**22.04.2026 Bulletin 2026/17**

(21) Application number: **26154570.1**

(22) Date of filing: **26.05.2022**

(51) International Patent Classification (IPC):
***G01N 33/569*** (2006.01)

(52) Cooperative Patent Classification (CPC):
**G01N 33/56994;** G01N 2333/045

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.05.2021 US 202163193529 P**
**25.05.2022 US 202263345811 P**

(62) Document number(s) of the earlier application(s) in accordance with Art. 76 EPC:
**22750745.6 / 4 348 257**

(71) Applicant: **GENENTECH, INC.**
**San Francisco, CA 94080-4990 (US)**

(72) Inventors:
• **KSCHONSAK, Marc**
**South San Francisco (US)**
• **GREEN, Evan Michael**
**South San Francisco (US)**
• **CIFERRI, Claudio**
**South San Francisco (US)**

(74) Representative: **Müller-Afraz, Simona**
**F. Hoffmann-La Roche AG**
**Patent Department**
**Grenzacherstrasse 124**
**4070 Basel (CH)**

Remarks:
•The complete document including Reference Table(s) and the Sequence Listing(s) can be downloaded from the EPO website
•This application was filed on 27-01-2026 as a divisional application to the application mentioned under INID code 62.

(54) **METHODS FOR MODULATING HOST CELL SURFACE INTERACTIONS WITH HUMAN CYTOMEGALOVIRUS**

(57) Provided herein are methods of treating or preventing human cytomegalovirus (HCMV) infection comprising modulating interactions between the HCMV gH/gL/UL128-131A pentamer and plasma membrane-expressed host cell proteins, as well as methods of identifying modulators of such interactions.

EP 4 729 942 A2

Fig. 1A

Fig. 1B

Fig. 1C

**(Cont. next page)**

# Fig. 1D

# Fig. 1E

# Fig. 1G

# Fig. 1F

# Fig. 1H

**Description**

**CROSS-REFERENCE TO RELATED APPLICATIONS**

This application claims priority to U.S. Patent Application No. 63/193,529, filed on May 26, 2021, and U.S. Patent Application No. 63/345,811, filed on May 25, 2022, the entire contents of which are incorporated herein by reference in their entirety.

**SEQUENCE LISTING**

The instant application contains a Sequence Listing which has been submitted electronically in ASCII format and is hereby incorporated by reference in its entirety. Said ASCII copy, created on May 25, 2022, is named 50474-270WO3_Sequence_Listing_5_25_22_ST25 and is 24,223 bytes in size.

**FIELD OF THE INVENTION**

Provided herein are methods of treating or preventing human cytomegalovirus (HCMV) infection comprising modulating interactions between the HCMV gH/gL/UL128-131A pentamer and plasma membrane-expressed host cell proteins, as well as methods of identifying modulators of such interactions.

**BACKGROUND**

Human cytomegalovirus (HCMV) is a member of the *Betaherpesvirinae* sub-family of *Herpesviridae* that establishes a life-long infection in more than 70% of the human population. After primary infection, HCMV becomes latent, and its reactivation causes severe morbidity and mortality in individuals who are immune-suppressed or undergoing organ or hematopoietic stem cell (HSC) transplantation. HCMV is particularly threatening during pregnancy due to its ability to cross the placental barrier and infect the fetus. HCMV infection affects 0.3% to 2.3% of newborns, representing the leading viral cause of congenital birth defects, including brain damage, hearing loss, learning disabilities, heart diseases and mental retardation. For these reasons, HCMV has been identified as a top priority disease target by the Institute of Medicine. An effective anti-viral therapeutic or vaccine should target the early steps of the HCMV infection cycle, including viral entry into host cells. HCMV uses several envelope glycoprotein complexes to enter different cell lines, including two gHgL envelope glycoprotein complexes, the gHgLgO (trimer) and the gHgLpUL128-131A (pentamer), as well as glycoprotein B (gB). HCMV trimer or pentamer binding to cellular host receptors provide the triggering signal, through a mechanism yet to be identified, for the HCMV glycoprotein gB to catalyze membrane fusion between the virus and infected cells. This fusion allows HCMV to enter cells, replicate, and establish its latency.

During the past decades, significant efforts have been established to develop vaccine candidates against HCMV infection. However, results from recent clinical trials indicated that HCMV vaccines showed only modest efficacy in preventing viral infection. Therefore, the development of effective therapeutics against HCMV represents an important unmet medical need.

**SUMMARY OF THE INVENTION**

In one aspect, the disclosure features a method of identifying a modulator of the interaction between the human cytomegalovirus (HCMV) gH/gL/UL128-131A pentamer and beta-2-microglobulin (B2M), the method comprising (a) providing a candidate modulator; (b) contacting the HCMV gH/gL/UL128-131A pentamer with B2M in the presence or absence of the candidate modulator under conditions permitting the binding of the HCMV gH/gL/UL128-131A pentamer to B2M; and (c) measuring the binding of the HCMV gH/gL/UL 128-131A pentamer to B2M, wherein an increase or decrease in binding in the presence of the candidate modulator relative to binding in the absence of the candidate modulator identifies the candidate modulator as a modulator of the interaction between the HCMV gH/gL/UL128-131A pentamer and B2M.

In another aspect, the disclosure features a method of identifying a modulator of a downstream activity of the HCMV gH/gL/UL128-131A pentamer, the method comprising (a) providing a candidate modulator; (b) contacting the HCMV gH/gL/UL128-131A pentamer with B2M in the presence or absence of the candidate modulator under conditions permitting the binding of the HCMV gH/gL/UL128-131A pentamer to B2M; and (c) measuring a downstream activity of the HCMV gH/gL/UL 128-131A pentamer, wherein a change in the downstream activity in the presence of the candidate modulator relative to the downstream activity in the absence of the candidate modulator identifies the candidate modulator as a modulator of the downstream activity of the HCMV gH/gL/UL 128-131A pentamer.

In another aspect, the disclosure features a method of identifying a modulator of a downstream activity of B2M, the method comprising (a) providing a candidate modulator; (b) contacting B2M with the HCMV gH/gL/UL128-131A pentamer in the presence or absence of the candidate modulator under conditions permitting the binding of B2M to the HCMV gH/gL/UL128-131A pentamer; and (c) measuring a downstream activity of B2M, wherein a change in the downstream activity in the presence of the candidate modulator relative to the downstream activity in the absence of the candidate modulator identifies the candidate modulator as a modulator of the downstream activity of B2M.

In some aspects, the increase or decrease in binding is at least 50%, as measured by surface plasmon resonance, biolayer interferometry, or an enzyme-linked immunosorbent assay (ELISA).

In some aspects, the modulator is an inhibitor of the downstream activity of the HCMV gH/gL/UL 128-131A pentamer or B2M.

In some aspects, the change in the downstream activity is a decrease in the amount, strength, or duration of the downstream activity.

In some aspects, the modulator is a small molecule, an antibody or antigen-binding fragment thereof, a peptide, a mimic, or an inhibitory nucleic acid. In some aspects, the inhibitory nucleic acid is an ASO or an siRNA. In some aspects, the antigen-binding fragment is a bis-Fab, an Fv, a Fab, a Fab'-SH, a F(ab')$_2$, a diabody, a linear antibody, an scFv, an scFab, a VH domain, or a VHH domain.

In some aspects, the antibody or antigen-binding fragment thereof binds the HCMV gH/gL/UL128-131A pentamer. In some aspects, the antibody or antigen-binding fragment thereof binds B2M.

In some aspects, the downstream activity is infection of a cell by HCMV. In some aspects, infection is decreased in the presence of the modulator. In some aspects, infection is decreased by at least 40%, as measured in a viral infection assay or a viral entry assay using pseudotyped particles.

In some aspects, the modulator is an antibody or antigen-binding fragment thereof that binds the HCMV gH/gL/UL128-131A pentamer. In some aspects, the modulator is an antibody or antigen-binding fragment thereof that binds B2M.

In another aspect, the disclosure features a modulator of the interaction between the human cytomegalovirus (HCMV) gH/gL/UL128-131A pentamer and neuropilin 2 (NRP2) that causes a decrease in the binding of the gH/gL/UL128-131A pentamer to NRP2, wherein the modulator binds to (a) one or more of residues D197, D252, N172, M253, Y458, and L459 of NRP2; (b) one or both of residues K47 and R57 of the UL128 subunit of the gH/gL/UL128-131A pentamer; (c) residue R193 of the UL130 subunit of the gH/gL/UL128-131A pentamer; and/or (d) one or both of residues A114 and A117 of the UL131A subunit of the gH/gL/UL128-131A pentamer.

In some aspects, the modulator binds to: (a) all six of residues D197, D252, N172, M253, Y458, and L459 of NRP2; (b) both of residues K47 and R57 of the UL128 subunit of the gH/gL/UL128-131A pentamer; (c) residue R193 of the UL130 subunit of the gH/gL/UL128-131A pentamer; and/or (d) both of residues A114 and A117 of the UL131A subunit of the gH/gL/UL128-131A pentamer.

In some aspects, the modulator decreases binding of the gH/gL/UL128-131A pentamer to NRP2 by at least 50%. In some aspects, the modulator decreases binding of the gH/gL/UL128-131A pentamer to NRP2 by at least 90%.

In another aspect, the disclosure features a modulator of the interaction between the HCMV gH/gL/UL128-131A pentamer and thrombomodulin (THBD) that causes a decrease in the binding of the gH/gL/UL128-131A pentamer to **THBD,** wherein the modulator binds to (a) one or more of residues S49, D53, V66, D69, R83, C96, E154, A123, L125, S149, and C133 of THBD; (b) one or more of residues R42, Y44, R131, N134, Y137, R158, R163, and Y168 of the UL128 subunit of the gH/gL/UL128-131A pentamer; and/or (c) one or more of residues N164, Y169, and M171 of the UL130 subunit of the gH/gL/UL128-131A pentamer.

In some aspects, the modulator binds to: (a) all eleven of residues S49, D53, V66, D69, R83, C96, E154, A123, L125, S149, and C133 of THBD; (b) all eight of residues R42, Y44, R131, N134, Y137, R158, R163, and Y168 of the UL128 subunit of the gH/gL/UL 128-131A pentamer; and/or (c) all three of residues N164, Y169, and M171 of the UL130 subunit of the gH/gL/UL 128-131A pentamer.

In some aspects, the modulator decreases binding of the gH/gL/UL128-131A pentamer to **THBD** by at least 50%. In some aspects, the modulator decreases binding of the gH/gL/UL128-131A pentamer to **THBD** by at least 90%.

In another aspect, the disclosure features a modulator of the interaction between the human cytomegalovirus (HCMV) gH/gL/UL128-131A pentamer and beta-2-microglobulin (B2M) that causes a decrease in the binding of the gH/gL/UL128-131A pentamer to B2M. In some aspects, the modulator decreases binding of the gH/gL/UL128-131A pentamer to B2M by at least 50%. In some aspects, the modulator decreases binding of the gH/gL/UL128-131A pentamer to THBD by at least 90%.

In some aspects, the decrease in binding is measured by surface plasmon resonance, biolayer interferometry, or an enzyme-linked immunosorbent assay (ELISA).

In some aspects, the modulator causes a decrease in infection of a cell by HCMV relative to infection in the absence of the modulator. In some aspects, infection is decreased by at least 40%, as measured in a viral infection assay or a viral entry assay using pseudotyped particles.

In some aspects, the modulator is a small molecule, an antibody or antigen-binding fragment thereof, a peptide, a mimic, or an inhibitory nucleic acid. In some aspects, the inhibitory nucleic acid is an antisense oligonucleotide (ASO) or an siRNA. In some aspects, the antigen-binding fragment is a bis-Fab, an Fv, a Fab, a Fab'-SH, a $F(ab')_2$, a diabody, a linear antibody, an scFv, an scFab, a VH domain, or a VHH domain.

In some aspects, the antibody is a bispecific antibody or a multispecific antibody.

In some aspects, the modulator further comprises a pharmaceutically acceptable carrier.

In another aspect, the disclosure features a method for treating an HCMV infection in an individual, the method comprising administering to the individual an effective amount of any one of the modulators provided herein, thereby treating the individual. In some aspects, the duration or severity of HCMV infection is decreased by at least 40% relative to an individual who has not been administered the modulator.

In another aspect, the disclosure features a method for preventing an HCMV infection in an individual, the method comprising administering to the individual an effective amount of any one of the modulators provided herein, thereby preventing an HCMV infection in the individual.

In another aspect, the disclosure features a method of prophylaxis against a secondary HCMV infection in an individual, the method comprising administering to the individual an effective amount of any one of the modulators provided herein, thereby preventing a secondary HCMV infection in the individual. In some aspects, the secondary infection is an HCMV infection of an uninfected tissue.

In some aspects, the individual is immunocompromised, is pregnant, or is an infant.

In another aspect, the disclosure features use of any one of the modulators provided herein in the manufacture of a medicament for treating an HCMV infection in an individual.

In another aspect, the disclosure features use of any one of the modulators provided herein in the manufacture of a medicament for preventing an HCMV infection in an individual.

In another aspect, the disclosure features use of any one of the modulators provided herein in the manufacture of a medicament for prophylaxis against a secondary HCMV infection in an individual. In some aspects, the secondary infection is an HCMV infection of an uninfected tissue.

In some aspects, the individual is immunocompromised, is pregnant, or is an infant.

In another aspect, the disclosure features any one of the modulators provided herein for use in a method of treating an HCMV infection in an individual, wherein the method comprises administering to the individual an effective amount of the modulator, thereby treating the individual.

In another aspect, the disclosure features any one of the modulators provided herein for use in a method of preventing an HCMV infection in an individual, wherein the method comprises administering to the individual an effective amount of the modulator, thereby preventing an HCMV infection in the individual.

In another aspect, the disclosure features any one of the modulators provided herein for use in a method of prophylaxis against a secondary HCMV infection in an individual, wherein the method comprises administering to the individual an effective amount of the modulator, thereby preventing a secondary HCMV infection in the individual. In some aspects, the secondary infection is an HCMV infection of an uninfected tissue.

In some aspects, the individual is immunocompromised, is pregnant, or is an infant.


## BRIEF DESCRIPTION OF THE DRAWINGS

**Fig. 1A** is a plot showing the normalized binding signal (percentage of maximum) of HCMV pentamer complexes encoded by the Merlin and VR1814 strains and neuropilin 1 (NRP1) and neuropilin 2 (NRP2) as detected using the cell-surface discovery platform described in Martinez-Martin et al., Cell. 174(5): 1158-1171.e19, 2018.

**Fig. 1B** is a schematic diagram showing the domain organization of human NRP2.

**Fig. 1C** is a diagram showing a front view of the HCMV pentamer complex (ribbon representation) bound to the NRP2 a2b1b2 domains (surface representation). Fabs are not rendered for clarity.

**Fig. 1D** is a diagram of the HCMV pentamer distal region showing the NRP2 a1a2b1b2 domains, UL128-131A, gL, and the gH N-terminus.

**Fig. 1E** is a close-up view of the HCMV pentamer distal region as described in Fig. 1D showing sites of interaction (Site 1) between the HCMV pentamer and the NRP2 a2 domain, including residues N172, M253, and D197 of NRP2 and residues R57 and K47 of UL128. Bolded residues represent reverse charge mutations.

**Fig. 1F** is a close-up view of the HCMV pentamer distal region as described in Fig. 1D showing sites of interaction (Site 2) between the HCMV pentamer and the NRP2 b2 domain, including residues Y458 and L459 of NRP2, residue R193 of UL130, and residues A114 and A1117 of UL131A. Bolded residues represent reverse charge mutations.

**Fig. 1G** is a close-up view (surface representation) of the HCMV pentamer distal region as described in Fig. 1D showing surface interaction areas with NRP1 (Sites 1 and 2).

**Fig. 1H** is a bar graph showing binding affinities of the HCMV pentamer to NRP2 variants comprising single point mutations (Site 1: N172R, M253E, A254E; Site 2: Y458R, L459R) relative to binding to wild-type (WT) NRP2. Kinetic parameters are representative of two independent assays.

**Fig. 2A** is a schematic diagram showing the domain organization of human thrombomodulin (THBD).

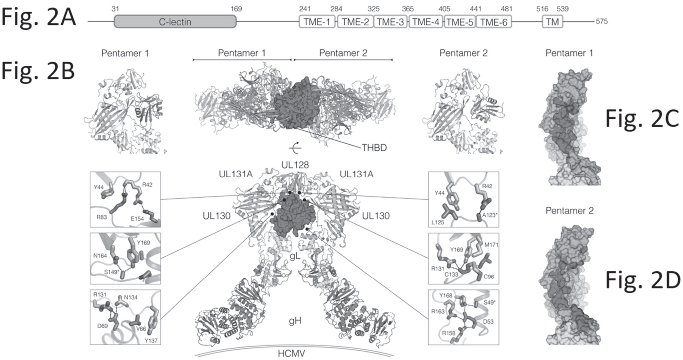

**Fig. 2B** is a set of diagrams showing front and top views of the HCMV pentamer complex (dimer comprising pentamer 1 and pentamer 2) (ribbon representation) bound to the THBD lectin domain (surface representation), including (a) a diagram of the HCMV pentamer 1 distal region showing the THBD lectin domains, UL128-131A, and gL with highlighted sites of interaction (inset panels) between pentamer 1 and the THBD lectin domain, including Y44, R42, R131, N134, and Y137 of UL128; R83, E154, S149, D69, and V66 of THBD; and N164 and Y169 of UL130 and (b) a diagram of the HCMV pentamer 2 distal region showing the THBD lectin domains, UL128-131A, and gL with highlighted sites of interaction (inset panels) between Pentamer 2 and the THBD lectin domain, including Y44, R42, R131, Y168, and R163 of UL128; L125, A123, C133, C96, S49, and D53 of THBD; and Y169 and M171 of UL130.

**Fig. 2C** is a close-up view (surface representation) of the HCMV pentamer 1 distal region as described in Fig. 3C showing surface interaction areas with THBD.

**Fig. 2D** is a close-up view (surface representation) of the HCMV pentamer 2 distal region as described in Fig. 3D showing surface interaction areas with THBD.

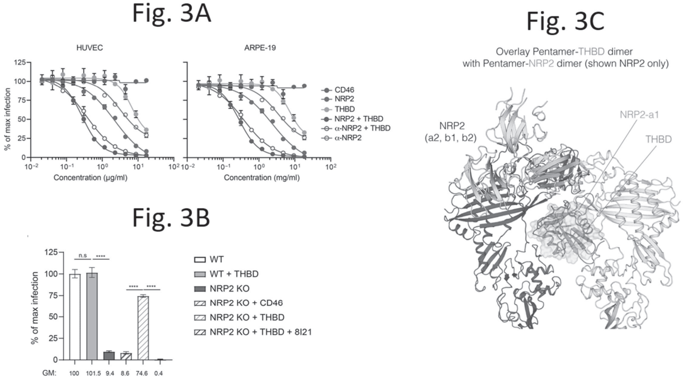

**Fig. 3A** is a pair of graphs showing the percent of infected cells in human umbilical vein endothelial cells (HUVEC) or ARPE-19 cells that were treated for 48 hours with HCMV strain VR1814 virus and that had been preincubated with the indicated concentrations of soluble recombinant CD46 (blue full circle), NRP2 (red full circle), an anti-NRP2 antibody (red open circle), THBD (orange full circle), a mix of recombinant NRP2 with THBD proteins (violet full circle), or a mix of anti-NRP2 with recombinant THBD proteins (violet full circle). The data shown are the means of three independent experiments $\pm$ SD.

**Fig. 3B** is a bar graph showing the percent of infected wild-type (WT) or NRP2 knockout (KO) HAP-1 cells in an assay in which cells were transduced with an empty lentivirus vector, a lentivirus vector encoding CD46, or lentivirus vector encoding THBD. NRP2 KO cells transduced with the lentivirus vector encoding THBD and treated with Fab 8121 are also shown. The percentage of maximum infection (geometric means of four independent experiments $\pm$ SD) is shown. n.s.: not significant.

**Fig. 3C** is a diagram showing an overlay of the HCMV pentamer in complex with NRP2 and the HCMV pentamer in complex with THBD. The NRP2-a1 domain is shown in surface representation.

**Fig. 3D** is a bar graph showing binding of the HCMV pentamer to wild-type (WT) NRP2-Fc in the presence of increasing concentrations of THBD (starting at equimolar ratio). Addition of 100-fold excess of transforming growth factor β receptor 3 (TGFβR3) is shown as a control.

**Fig. 3E** is a diagram showing an overlay of the HCMV pentamer-NRP2 dimer cryo-EM map and the dimeric structure of HCMV pentamer-THBD.

**Fig. 3F** is a diagram showing the HCMV pentamer dimerization interface mediated by UL128.

**Fig. 3G** is a diagram showing a close-up top view of the dimeric interaction interface mediated by UL128 (as shown in Fig. 3F).

**Fig. 4A** is a is a diagram showing a front view of a composite structure of the HCMV pentamer complex (ribbon representation) bound to the pentamer-specific neutralizing antibodies 2C12, 7I13 and 8I21 (shown in surface representation) and the gH-specific neutralizing antibodies 13H11 and MSL-109 (shown in surface representation).

**Fig. 4B** is a close-up view of the HCMV pentamer distal region showing the pentamer-specific neutralizing antibody 2C12, UL128-131A, and gL with highlighted sites of interaction between the pentamer and 2C12.

**Fig. 4C** is a close-up view of the HCMV pentamer distal region showing the pentamer-specific neutralizing antibody 2C12, UL128-131A, and gL with highlighted sites of interaction between the pentamer and 2C12.

**Fig. 4D** is a close-up view of the HCMV pentamer distal region showing the highlighted interaction interface between the pentamer and 2C12 (left) or 7I13 (right).

**Fig. 4E** is a close-up view of the HCMV pentamer distal region showing 2C12, UL128-131A, and gL 2C12 and 7I13.

**Fig. 4F** is a close-up view of the HCMV pentamer distal region showing 7I13, UL128-131A, and gL with highlighted sites of interaction between the pentamer and 7I13.

**Fig. 4G** is a close-up view of the HCMV pentamer distal region showing 7I13, UL128-131A, and gL with highlighted sites of interaction between the pentamer and 7I13.

**Fig. 4H** is a close-up view of the HCMV pentamer distal region showing 7I13, UL128-131A, and gL with highlighted sites of interaction between the pentamer and 7I13.

**Fig. 4I** is a close-up view of the HCMV pentamer distal region showing 7I13, UL128-131A, and gL with highlighted sites of interaction between the pentamer and 7I13.

**Fig. 4J** is a diagram showing an overlay of the HCMV pentamer bound to NRP2 and the HCMV pentamer bound to 2C12.

**Fig. 4K** is a diagram showing an overlay of the HCMV pentamer bound to THBD and the HCMV pentamer bound to 7I13.

**Fig. 4L** is a bar graph showing binding of the HCMV pentamer to WT NRP2-Fc in the presence of the indicated neutralizing Fabs (2C12, 7113, 8I21, MSL-109, and 13H11).

**Fig. 4M** is a bar graph showing binding of the HCMV pentamer to WT THBD-Fc in the presence of the indicated neutralizing Fabs (2C12, 7113, 8I21, MSL-109, and 13H11).

**Fig.** 5 is a diagram showing a model of HCMV pentamer-mediated entry of HCMV into epithelial and endothelial host cells. Receptor binding and possibly pentamer dimerization could mediate receptor clustering and facilitate a high-affinity and stable tethering of the viral membrane with the host cell membrane. Membrane tethering would trigger the transition of gB from its prefusion to post-fusion conformation and initiate membrane fusion.

**Fig. 6A** is a diagram showing a workflow for purification and reconstitution of the HCMV pentamer bound to NRP2 and the Fabs 13H11 and 8I21.

**Fig. 6B** is a graph and a gel image showing results of a size exclusion chromatography (SEC) assay of the HCMV pentamer bound to NRP2 and the Fabs 13H11 and 8121.

**Fig. 6C** is a representative cryoEM micrograph showing the HCMV pentamer bound to NRP2 and 13H11 and 8I21.

**Fig. 6D** is a set of images showing representative 2D class averages of monomeric and dimeric HCMV pentamer-NRP2 bound to 13H11 and 8I21.

**Fig. 6E** is a diagram showing a processing workflow to obtain an ab-initio 3D reconstruction of monomeric and dimeric HCMV pentamer-NRP2 bound to 13H11 and 8121.

**Fig. 6F** is a diagram showing a data collection and processing workflow to obtain a 3D reconstruction of dimeric HCMV pentamer-NRP2 bound to 13H11 and 8121.

**Fig. 6G** is a diagram showing a data collection and processing workflow to obtain a high-resolution 3D reconstruction of dimeric HCMV pentamer-NRP2 bound to 13H11 and 8121. The reconstruction is shown in Fig. 6H.

**Fig. 6H** is a heatmap showing the distribution of the assigned particle orientations determined in the workflow of Fig. 6G and a high-resolution 3D reconstruction of the dimeric HCMV pentamer-NRP2 bound to 13H11 and 8I21.

**Fig. 6I** is a graph showing the Fourier Shell Correlation (FSC) between half datasets for the overall 3D reconstruction of the HCMV pentamer-NRP2 bound to Fabs 13H11 and 8I21 and for the focused refinement reconstruction (as shown in Fig. 6G).

**Fig. 7A** is an exemplary 3D map overlay for NRP2 bound to the HCMV pentamer.

**Fig. 7B** is an exemplary 3D map overlay for THBD bound to the HCMV pentamer.

**Fig. 7C** is an exemplary 3D map overlay for the Fab 2C12 bound to the HCMV pentamer.

**Fig. 7D** is an exemplary 3D map overlay for the Fab 7113 bound to the HCMV pentamer.

**Fig. 8A** is a set of diagrams showing superposition of the a2b1b2 domains of the cryoEM HCMV pentamer-NRP2 with the NRP2 crystal structure (PDB: 2QQORMSD 0.6 Å/328 C$\alpha$) and the NRP1 crystal structure (2QQN, RMSD 2.0Å/316 C$\alpha$).

**Fig. 8B** is a pair of diagrams showing the superposition of the NRP2 a2 and b2 domains of the cryoEM HCMV Pentamer-NRP2 with the NRP1 crystal structure (2QQN, a2: RMSD 0.7Å/98 C$\alpha$; b2: RMSD 0.8Å/122 C$\alpha$).

**Fig. 8C** is a diagram showing a sequence alignment of the a1a2b1b2 domains of NRP1 (SEQ ID NO: 6) and NRP2 (SEQ ID NO: 7).

**Fig. 8D** is a set of diagrams showing a comparison between the HCMV pentamer-NRP2 interaction involving the a2 and b2 domains and the canonical binding site of NRP2 b1-VEGFC peptide (PDB: 6TJT) and NRP1 b1- SARS-CoV-2 (PDB:7JJC).

**Fig. 9A** is a diagram showing the gHgL subunits of the HCMV pentamer complex (blue/pink) superimposed onto the gHgL subunits of the HCMV trimer complex (orange, PDB: 7LBE (Kschonsak et al., Cell, 184: 1232-1244.e16, 2021)); root-mean-square deviation (RMSD) 0.5 Å/765 C$\alpha$.

**Fig. 9B** is a diagram showing the cryoEM HCMV pentamer-13H11-2C12-7I13 complex (blue/pink) superimposed onto the X-ray HCMV pentamer complex (green, PDB: 5VOB; Chandramouli et al., Sci. Immunol., 2: 2017; RMSD 1.2 Å/980 C$\alpha$).

**Fig. 9C** is a diagram showing the HCMV pentamer-13H11-2C12-7I13 complex superimposed onto the HCMV pentamer-NRP2-13H11-8I21 complex (yellow, RMSD 0.6 Å/937 C$\alpha$).

**Fig. 9D** is a pair of diagrams showing the HCMV pentamer-13H11-2C12-7I13 complex superimposed onto both HCMV pentamer-THBD-13H11 molecules (purple/magenta, (1) RMSD 0.6 Å/846 C$\alpha$ (2) RMSD 0.6 Å/622 C$\alpha$). **Fig. 10A** is a graph and a gel image showing results of a SEC assay of the HCMV pentamer-THBD complex bound to the Fabs 13H11 and MSL-109.

**Fig. 10B** is a representative cryoEM micrograph showing the HCMV pentamer bound to THBD and the Fabs 13H11 and MSL-109.

**Fig. 10C** is a set of images showing representative 2D class averages of monomeric and dimeric HCMV pentamer-THBD bound to 13H11 and MSL-109.

**Fig. 10D** is a diagram showing a processing workflow to obtain an ab-initio and high-resolution 3D reconstruction of monomeric and dimeric HCMV pentamer-THBD bound to Fabs 13H11 and MSL-109. The reconstruction is shown in Fig. 10E.

**Fig. 10E** is a heatmap showing the distribution of the assigned particle orientations determined in the workflow of Fig. 10D and a high-resolution 3D reconstruction of the HCMV pentamer-THBD bound to Fabs 13H11 and MSL-109.

**Fig. 10F** is a graph showing the FSC between half datasets for the overall monomeric pentamer-THBD bound to Fabs 13H11 and MSL-109 3D reconstruction and for the focused refinement reconstructions (as shown in Fig. 10D).

**Fig. 11A** is a diagram showing the HCMV pentamer complex bound to THBD. The THBD TME-1 domain and the linker region located between the N-terminal Lectin domain and the TME-1 domain are shown.

**Fig. 11B** is a diagram showing the structure of the THBD lectin domain. Secondary structure organization is shown.

**Fig. 12A** is a set of graphs showing cell surface staining (as percent of maximum) of NRP2, CD46, and THBD on WT or NRP2 KO HAP-1 cells. Blue line: staining with isotype control antibody; green line: staining with a target-specific antibody. Flow cytometry staining was repeated three times (n=10,000 cells).

**Fig. 12B** is a bar graph showing the percent of infected WT, NRP2 KO, or NRP2 KO + THBD HAP-1 cells in an assay in which cells were treated with VR1814 to obtain low numbers of infected cells (MOI of 0.1) and virus spread was monitored at day 8 by staining for HCMV pp72 protein.

**Fig. 12C** is a set of micrographs showing representative images of infected cells of Fig. 12A stained with anti- pp72. Scale bar is 10 micrometers.

**Fig. 13A** is a diagram showing a workflow for purification and reconstitution of the HCMV pentamer bound to the Fabs 13H11, 2C12, and 7I13.

**Fig. 13B** is a graph and a gel image showing results of a SEC assay of the HCMV pentamer bound to 13H11, 2C12, and 7I13.

**Fig. 13C** is a representative cryoEM micrograph showing the HCMV pentamer bound to 13H11, 2C12, and 7I13.

**Fig. 13D** is a set of images showing representative 2D class averages of the HCMV pentamer bound to 13H11, 2C12, and 7I13.

**Fig. 13E** is a diagram showing a processing workflow to obtain an ab-initio 3D reconstruction of the HCMV pentamer bound to 13H11, 2C12, and 7I13.

**Fig. 13F** is a diagram showing a data collection and processing workflow to obtain a high-resolution 3D reconstruction of the HCMV pentamer bound to 13H11, 2C12, and 7I13. The reconstruction is shown in Fig. 8G.

**Fig. 13G** is a heatmap showing the distribution of the assigned particle orientations determined in the workflow of Fig. 8F and a high-resolution 3D reconstruction of the HCMV pentamer bound to 13H11, 2C12, and 7I13.

**Fig. 13H** is a FSC between half datasets for the overall 3D reconstruction of the HCMV pentamer bound to 13H11, 2C12, and 7I13 and for the focused refinement reconstructions (as shown in Fig. 8F).

**Fig. 14A** is a diagram showing superposition of the cryoEM HCMV Pentamer-8I21 complex (blue/orange/pink) onto the X-ray HCMV pentamer-8I21 complex (green, PDB: 5VOB RMSD 1.3 A/477 C$\alpha$).

**Fig. 14B** is a diagram showing an overlay of the HCMV pentamer-NRP2 complex and a composite structure of the HCMV pentamer bound to 2C12, 7I13, and 8I21.

**Fig. 14C** is a diagram showing an overlay of the HCMV pentamer-THBD complex and a composite structure of the HCMV pentamer bound to 2C12, 7I13, and 8I21.

**Fig. 15A** is a diagram showing a front view of the HCMV pentamer complex (ribbon representation) bound to beta-2-microglobulin (B2M) (surface representation).

**Fig. 15B** is a diagram of the HCMV pentamer distal region showing B2M, UL128-131A, gL, and the N-terminal region of gH.

**Fig. 15C** is a close-up view (surface representation) of the HCMV pentamer distal region showing surface interaction areas with B2M.

**Fig. 15D** is a diagram showing an overlay of the THBD lectin domain and B2M interacting on the concave region of the UL subunits of the HCMV pentamer.

**Fig. 15E** is a diagram showing an overlay of B2M-bound HCMV pentamer and a B2M MHC class 1 complex (Protein Data Bank (PDB): 1A1M).

**Fig. 16A** is a graph showing results of a SEC assay of the HCMV pentamer mixed with olfactory receptor (OR14L1) (1-26)-EGFP.

**Fig. 16B** is an SDS-PAGE blot and the output of a mass spectrometry (MS) analysis showing identification of B2M as an HCMV pentamer binding partner.

**Fig. 16C** is a representative cryoEM micrograph of Pentamer-B2M bound to the Fabs 13H11 and MSL-109.

**Fig. 16D** is a set of images showing representative 2D class averages of pentamer-B2M bound to the Fabs 13H11 and MSL-109.

**Fig. 16E** is a schematic diagram showing a processing workflow to obtain an ab-initio and high-resolution 3D reconstruction of pentamer-B2M bound to the Fabs 13H11 and MSL-109.

**Fig. 16F** is a heatmap representation of the distribution of assigned particle orientations determined in the workflow of Fig. 16E.

**Fig. 16G** is a graph showing the FSC between half datasets for the overall pentamer-B2M bound to Fabs 13H11 and MSL-109 3D reconstruction

## DETAILED DESCRIPTION OF THE INVENTION

### I. DEFINITIONS

Unless otherwise defined, all terms of art, notations, and other scientific terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art.

The term "about" as used herein refers to the usual error range for the respective value readily known to the skilled person in this technical field. Reference to "about" a value or parameter herein includes (and describes) aspects that are directed to that value or parameter *per se.*

As used herein, the singular forms "a," "an," and "the" include plural referents unless the context clearly dictates otherwise. For example, reference to "an isolated peptide" means one or more isolated peptides.

Throughout this specification and claims, the word "comprise," or variations such as "comprises" or "comprising" will be understood to imply the inclusion of a stated integer or group of integers but not the exclusion of any other integer or group of integers.

The terms "patient," "subject," or "individual," as used interchangeably herein, refer to a human patient.

An "intravenous" or "iv" dose, administration, or formulation of a drug is one which is administered via a vein, e.g. by infusion.

A "subcutaneous" or "sc" dose, administration, or formulation of a drug is one which is administered under the skin, e.g. via a pre-filled syringe, auto-injector, or other device.

For the purposes herein, "clinical status" refers to a patient's health condition. Examples include that the patient is improving or getting worse. In one embodiment, clinical status is based on an ordinal scale of clinical status. In one embodiment, clinical status is not based on whether or not the patient has a fever.

An "effective amount" refers to an amount of an agent (e.g., a therapeutic agent) that is effective to bring about a therapeutic/prophylactic benefit (e.g., as described herein) that is not outweighed by unwanted/undesirable side effects.

The term "pharmaceutical formulation" refers to a preparation which is in such form as to permit the biological activity of the active ingredient or ingredients to be effective, and which contains no additional components which are unacceptably toxic to a subject to which the formulation would be administered. Such formulations are sterile. In one embodiment, the formulation is for intravenous (iv) administration. In another embodiment, the formulation is for subcutaneous (sc) administration.

A "native sequence" protein herein refers to a protein comprising the amino acid sequence of a protein found in nature, including naturally occurring variants of the protein. The term as used herein includes the protein as isolated from a natural source thereof or as recombinantly produced.

The term "protein," as used herein, refers to any native protein from any vertebrate source, including mammals such as primates (e.g., humans) and rodents (e.g., mice and rats), unless otherwise indicated. The term encompasses "full-length," unprocessed protein any form of the protein that results from processing in the cell. The term also encompasses naturally occurring variants of the protein, e.g., splice variants or allelic variants, e.g., amino acid substitution mutations or amino acid deletion mutations. The term also includes isolated regions or domains of the protein, e.g., the extracellular domain (ECD).

An "isolated" protein or peptide is one which has been separated from a component of its natural environment. In some aspects, a protein or peptide is purified to greater than 95% or 99% purity as determined by, for example, electrophoresis (e.g., SDS-PAGE, isoelectric focusing (IEF), capillary electrophoresis) or chromatography (e.g., ion exchange or reverse phase HPLC).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

As used herein, the terms "human cytomegalovirus (HCMV) pentamer," "HCMV gH/gL/UL128-131A pentamer," and "HCMV pentamer" refer to a glycoprotein complex that is located on the outer surface of the viral envelope of human cytomegalovirus (HCMV) and is composed of gH, gL, UL128, UL130, and UL131A glycoprotein subunits.

The terms "gH subunit of human cytomegalovirus (HCMV)," "gH subunit," and "gH," as used herein, broadly refer to any native gH from any viral source, unless otherwise indicated. The term encompasses full-length gH and isolated regions or domains of gH. The term also encompasses naturally occurring variants of gH, e.g., splice variants or allelic variants. The amino acid sequence of an exemplary HCMV gH is provided as SEQ ID NO: 1. Minor sequence variations, especially conservative amino acid substitutions of gH that do not affect gH function and/or activity, are also contemplated by the invention.

The terms "gL subunit of human cytomegalovirus (HCMV)," "gL subunit," and "gL," as used herein, broadly refer to any native gL from any viral source, unless otherwise indicated. The term encompasses full-length gL and isolated regions or domains of gL. The term also encompasses naturally occurring variants of gL, e.g., splice variants or allelic variants. The amino acid sequence of an exemplary HCMV gL is provided as SEQ ID NO: 2. Minor sequence variations, especially conservative amino acid substitutions of gL that do not affect gL function and/or activity, are also contemplated by the invention.

The terms "UL128 subunit of human cytomegalovirus (HCMV)," "UL128 subunit," and "UL128," as used herein, broadly refer to any native UL128 from any viral source, unless otherwise indicated. The term encompasses full-length UL128 and isolated regions or domains of UL128. The term also encompasses naturally occurring variants of UL128, e.g., splice variants or allelic variants. The amino acid sequence of an exemplary HCMV UL128 is provided as SEQ ID NO: 3. Minor sequence variations, especially conservative amino acid substitutions of UL128 that do not affect UL128 function and/or activity, are also contemplated by the invention.

The terms "UL130 subunit of human cytomegalovirus (HCMV)," "UL130 subunit," and "UL130," as used herein, broadly refer to any native UL130 from any viral source, unless otherwise indicated. The term encompasses full-length UL130 and isolated regions or domains of UL130. The term also encompasses naturally occurring variants of UL130, e.g., splice variants or allelic variants. The amino acid sequence of an exemplary HCMV UL130 is provided as SEQ ID NO: 4. Minor sequence variations, especially conservative amino acid substitutions of UL130 that do not affect UL130 function and/or activity, are also contemplated by the invention.

The terms "UL131A subunit of human cytomegalovirus (HCMV)," "UL131A subunit," and "UL131A," as used herein, broadly refer to any native UL131A from any viral source, unless otherwise indicated. The term encompasses full-length UL131A and isolated regions or domains of UL131A. The term also encompasses naturally occurring variants of UL131A, e.g., splice variants or allelic variants. The amino acid sequence of an exemplary HCMV UL131A is provided as SEQ ID NO: 5. Minor sequence variations, especially conservative amino acid substitutions of UL131A that do not affect UL131A function and/or activity, are also contemplated by the invention.

As used herein, a "modulator" is an agent that modulates (e.g., increases, decreases, activates, or inhibits) a given biological activity, e.g., an interaction or a downstream activity resulting from an interaction. A modulator or candidate modulator may be, e.g., a small molecule, an antibody (e.g., a bispecific or multispecific antibody), an antigen-binding fragment (e.g., a bis-Fab, an Fv, a Fab, a Fab'-SH, a $F(ab')_2$, a diabody, a linear antibody, an scFv, an ScFab, a VH domain, or a VHH domain), a peptide, a mimic, an antisense oligonucleotide, or an inhibitory nucleic acid (e.g., an antisense oligonucleotide (ASO) or a small interfering RNA (siRNA)).

By "increase" or "activate" is meant the ability to cause an overall increase, for example, of 20% or greater, of 50% or greater, or of 75%, 85%, 90%, or 95% or greater. In certain aspects, increase or activate can refer to a downstream activity of a protein-protein interaction.

By "reduce" or "inhibit" is meant the ability to cause an overall decrease, for example, of 20% or greater, of 50% or greater, or of 75%, 85%, 90%, or 95% or greater. In certain aspects, reduce or inhibit can refer to a downstream activity of a protein-protein interaction.

"Affinity" refers to the strength of the sum total of noncovalent interactions between a single binding site of a molecule (e.g., a receptor) and its binding partner (e.g., a ligand). Unless indicated otherwise, as used herein, "binding affinity" refers to intrinsic binding affinity, which reflects a 1:1 interaction between members of a binding pair (e.g., receptor and ligand). The affinity of a molecule X for its partner Y can generally be represented by the dissociation constant ($K_D$). Affinity can be measured by common methods known in the art, including those described herein.

"Complex" or "complexed" as used herein refers to the association of two or more molecules that interact with each other through bonds and/or forces (e.g., Van der Waals, hydrophobic, hydrophilic forces) that are not peptide bonds. In one aspect, a complex is heteromultimeric. It should be understood that the term "protein complex" or "polypeptide complex" as used herein includes complexes that have a non-protein entity conjugated to a protein in the protein complex (e.g., including, but not limited to, chemical molecules such as a toxin or a detection agent).

The terms "host cell," "host cell line," and "host cell culture" are used interchangeably and refer to cells into which exogenous nucleic acid has been introduced, including the progeny of such cells. Host cells include "transfected cells," "transformed cells," and "transformants," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. In some aspects, the host cell is stably transformed with the exogenous nucleic acid. In other aspects, the host cell is transiently transformed with the exogenous nucleic acid.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The term "antibody" herein is used in the broadest sense and encompasses various antibody structures, including but not limited to monoclonal antibodies, polyclonal antibodies, multispecific antibodies (e.g., bispecific antibodies), and antibody fragments (e.g., bis-Fabs) so long as they exhibit the desired antigen-binding activity.

An "antigen-binding fragment" or "antibody fragment" refers to a molecule other than an intact antibody that comprises a portion of an intact antibody that binds the antigen to which the intact antibody binds. Examples of antigen-binding fragments include but are not limited to bis-Fabs; Fv; Fab, Fab, Fab'-SH; $F(ab')_2$; diabodies; linear antibodies; single-chain antibody molecules (e.g., scFv, scFab); and multispecific antibodies formed from antibody fragments.

A "single-domain antibody" refers to an antibody fragment comprising all or a portion of the heavy chain variable domain or all or a portion of the light chain variable domain of an antibody. In certain aspects, a single-domain antibody is a human single-domain antibody (see, e.g., U.S. Patent No. 6,248,516 B1). Examples of single-domain antibodies include but are not limited to a VHH.

A "Fab" fragment is an antigen-binding fragment generated by papain digestion of antibodies and consists of an entire L chain along with the variable region domain of the H chain (VH), and the first constant domain of one heavy chain (CH1). Papain digestion of antibodies produces two identical Fab fragments. Pepsin treatment of an antibody yields a single large $F(ab')_2$ fragment which roughly corresponds to two disulfide linked Fab fragments having divalent antigen-binding activity and is still capable of cross-linking antigen. Fab' fragments differ from Fab fragments by having an additional few residues at the carboxy terminus of the CH1 domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments which have hinge cysteines between them. Other chemical couplings of antibody fragments are also known.

The term "Fc region" herein is used to define a C-terminal region of an immunoglobulin heavy chain, including native sequence Fc regions and variant Fc regions. Although the boundaries of the Fc region of an immunoglobulin heavy chain might vary, the human IgG heavy chain Fc region is usually defined to stretch from an amino acid residue at position Cys226, or from Pro230, to the carboxyl- terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all Lys447 residues removed, antibody populations with no Lys447 residues removed, and antibody populations having a mixture of antibodies with and without the Lys447 residue.

"Fv" consists of a dimer of one heavy- and one light-chain variable region domain in tight, noncovalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three CDRs specific for an antigen) has the ability to recognize and bind antigen, although often at a lower affinity than the entire binding site.

The terms "full-length antibody," "intact antibody," and "whole antibody" are used herein interchangeably to refer to an antibody having a structure substantially similar to a native antibody structure or having heavy chains that contain an Fc region as defined herein.

"Single-chain Fv" also abbreviated as "sFv" or "scFv" are antibody fragments that comprise the VH and VL antibody domains connected into a single polypeptide chain. Preferably, the scFv polypeptide further comprises a polypeptide linker between the VH and VL domains, which enables the scFv to form the desired structure for antigen binding. For a review of scFv, see Pluckthun, The Pharmacology of Monoclonal Antibodies, vol. 113, Rosenburg and Moore eds., Springer-Verlag, New York, pp. 269-315 (1994); Malmborg et al., J. Immunol. Methods 183:7-13, 1995.

The term "small molecule" refers to any molecule with a molecular weight of about 2000 daltons or less, e.g., about 1000 daltons or less. In some aspects, the small molecule is a small organic molecule.

The term "mimic" or "molecular mimic," as used herein, refers to a polypeptide having sufficient similarity in conformation and/or binding ability (e.g., secondary structure, tertiary structure) to a given polypeptide or to a portion of said polypeptide to bind to a binding partner of said polypeptide. The mimic may bind the binding partner with equal, less, or greater affinity than the polypeptide it mimics. A molecular mimic may or may not have obvious amino acid sequence similarity to the polypeptide it mimics. A mimic may be naturally occurring or may be engineered. In some aspects, the mimic is a mimic of a member of a binding pair. In yet other aspects, the mimic is a mimic of another protein that binds to a member of the binding pair. In some aspects, the mimic may perform all functions of the mimicked polypeptide. In other aspects, the mimic does not perform all functions of the mimicked polypeptide.

As used herein, the term "conditions permitting the binding" of two or more proteins to each other refers to conditions (e.g., protein concentration, temperature, pH, salt concentration) under which the two or more proteins would interact in the absence of a modulator or a candidate modulator. Conditions permitting binding may differ for individual proteins and may differ between protein-protein interaction assays (e.g., surface plasmon resonance assays, biolayer interferometry assays, enzyme-linked immunosorbent assays (ELISA), extracellular interaction assays, and cell surface interaction assays.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full-length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease (e.g., preventing HCMV infection or symptoms thereof), reducing or preventing secondary infection in a patient having an infection (e.g., reducing or preventing secondary infection of nervous tissue, immune cells, lymphoid tissue, and/or lung tissue), alleviation of symptoms, diminishment of any direct or indirect pathological consequences of the disease, decreasing the rate of disease progression, amelioration or palliation of the disease state, and remission or improved prognosis.

The "pathology" of a disease or condition includes all phenomena that compromise the well-being of the patient.

"Amelioration," "ameliorating," "alleviation," "alleviating," or equivalents thereof, refers to both therapeutic treatment and prophylactic or preventative measures, wherein the object is to ameliorate, prevent, slow down (lessen), decrease or inhibit a disease or condition, e.g., HCMV infection. Those in need of treatment include those already with the disease or condition as well as those prone to having the disease or condition or those in whom the disease or condition is to be prevented.

## II. MODULATORS OF PROTEIN-PROTEIN INTERACTIONS

In some aspects, the disclosure features an isolated modulator of the interaction between neuropilin 2 (NRP2), thrombomodulin (THBD), or beta-2-microglobulin (B2M) and the human cytomegalovirus (HCMV) gH/gL/UL128-131A pentamer, wherein the modulator causes a decrease in the binding of the HCMV gH/gL/UL128-131A pentamer to NRP2, THBD, or B2M relative to binding in the absence of the modulator.

In some aspects, the modulator comprises a pharmaceutically acceptable carrier.

### A. Modulators of the interaction between NRP2 and the HCMV gH/gL/UL128-131A pentamer

In some aspects, the disclosure features a modulator of the interaction between the HCMV gH/gL/UL 128-131A pentamer and neuropilin 2 (NRP2) that causes a decrease in the binding of the gH/gL/UL128-131A pentamer to NRP2, wherein the modulator binds to: (a) one or more of residues D197, D252, N172, M253, Y458, and L459 of NRP2 (e.g., one, two, three, four, five, or all six of residues D197, D252, N172, M253, Y458, and L459); (b) one or both of residues K47 and R57 of the UL128 subunit of the gH/gL/UL128-131A pentamer (e.g., one or both of residues K47 and R57); (c) residue R193 of the UL130 subunit of the gH/gL/UL128-131A pentamer; and/or (d) one or both of residues A114 and A117 of the UL131A subunit of the gH/gL/UL128-131A pentamer.

In some aspects, the modulator binds to: (a) all six of residues D197, D252, N172, M253, Y458, and L459 of NRP2; (b) both of residues K47 and R57 of the UL128 subunit of the gH/gL/UL128-131A pentamer; (c) residue R193 of the UL130 subunit of the gH/gL/UL128-131A pentamer; and/or (d) both of residues A114 and A117 of the UL131A subunit of the gH/gL/UL128-131A pentamer.

In some aspects, the modulator binds to: (a) all six of residues D197, D252, N172, M253, Y458, and L459 of NRP2; (b) both of residues K47 and R57 of the UL128 subunit of the gH/gL/UL128-131A pentamer; (c) residue R193 of the UL130 subunit of the gH/gL/UL128-131A pentamer; and (d) both of residues A114 and A117 of the UL131A subunit of the gH/gL/UL128-131A pentamer.

In some aspects, the modulator decreases binding of the gH/gL/UL128-131A pentamer to NRP2 by at least 50%. In some aspects, the decrease in binding is at least 5%, 10%, 15%, 20%, 25%, 30%, 35%, 40%, 45%, 50%, 55%, 60%, 65%, 70%, 75%, 80%, 85%, 90%, 95%, 98%, or 99% or is 100% (i.e., binding is abolished), e.g., the decrease is 5%-15%, 15%-25%, 25%-35%, 35%-45%, 45%-55%, 55%-65%, 65%-75%, 75%-85%, 85%-95%, or 95%-100%, relative to binding in the absence of the modulator. In some aspects, the modulator decreases binding of the gH/gL/UL128-131A pentamer to NRP2 by at least 90% (e.g., 90%-100%). In some aspects, the decrease in binding is at least 50% (e.g., is 50%-55%, 55%-65%, 65%-75%, 75%-85%, 85%-95%, or 95%-100%), e.g., as measured by surface plasmon resonance, biolayer interferometry, or an enzyme-linked immunosorbent assay (ELISA).

In some aspects, the modulator causes a decrease in infection of a cell by HCMV relative to infection in the absence of the modulator. In some aspects, infection is decreased by at least 40%, as measured in a viral infection assay or a viral entry assay using pseudotyped particles. In some aspects, the decrease is at least 5%, 10%, 15%, 20%, 25%, 30%, 35%, 40%, 45%, 50%, 55%, 60%, 65%, 70%, 75%, 80%, 85%, 90%, 95%, 98%, or 99% or is 100% (i.e., no infection occurs), e.g., the decrease is 5%-15%, 15%-25%, 25%-35%, 35%-45%, 45%-55%, 55%-65%, 65%-75%, 75%-85%, 85%-95%, or 95%-100%).

### *B. Modulators of the interaction between THBD and the HCMV* gH/gL/UL128-131A *pentamer*

In some aspects, the disclosure features a modulator of the interaction between the HCMV gH/gL/UL128-131A pentamer and thrombomodulin (THBD) that causes a decrease in the binding of the gH/gL/UL128-131A pentamer to THBD, wherein the modulator binds to: (a) one or more of residues S49, D53, V66, D69, R83, C96, E154, A123, L125, S149, and C133 of THBD (e.g., one, two, three, four, five, six, seven, eight, nine, ten, or all eleven of residues S49, D53, V66, D69, R83, C96, E154, A123, L125, S149, and C133); (b) one or more of residues R42, Y44, R131, N134, Y137, R158, R163, and Y168 of the UL128 subunit of the gH/gL/UL128-131A pentamer (e.g., one, two, three, four, five, six, seven, or all eight of residues R42, Y44, R131, N134, Y137, R158, R163, and Y168); and/or (c) one or more of residues N164, Y169, and M171 of the UL130 subunit of the gH/gL/UL128-131A pentamer (e.g., one, two, or all three of residues N164, Y169, and M171).

In some aspects, the modulator binds to (a) all eleven of residues S49, D53, V66, D69, R83, C96, E154, A123, L125, S149, and C133 of THBD; (b) all eight of residues R42, Y44, R131, N134, Y137, R158, R163, and Y168 of the UL128 subunit of the gH/gL/UL128-131A pentamer; and/or (c) all three of residues N164, Y169, and M171 of the UL130 subunit of the gH/gL/UL128-131A pentamer.

In some aspects, the modulator binds to (a) all eleven of residues S49, D53, V66, D69, R83, C96, E154, A123, L125, S149, and C133 of THBD; (b) all eight of residues R42, Y44, R131, N134, Y137, R158, R163, and Y168 of the UL128 subunit of the gH/gL/UL128-131A pentamer; and (c) all three of residues N164, Y169, and M171 of the UL130 subunit of the gH/gL/UL128-131A pentamer.

In some aspects, the modulator decreases binding of the gO subunit of the gH/gL/UL128-131A pentamer to THBD by at least 50%. In some aspects, the decrease in binding is at least 5%, 10%, 15%, 20%, 25%, 30%, 35%, 40%, 45%, 50%, 55%, 60%, 65%, 70%, 75%, 80%, 85%, 90%, 95%, 98%, or 99% or is 100% (i.e., binding is abolished), e.g., the decrease is 5%-15%, 15%-25%, 25%-35%, 35%-45%, 45%-55%, 55%-65%, 65%-75%, 75%-85%, 85%-95%, or 95%-100%, relative to binding in the absence of the modulator. In some aspects, the modulator decreases binding of the gH/gL/UL128-131A pentamer to THBD by at least 90% (e.g., 90%-100%). In some aspects, the decrease in binding is at least 50% (e.g., is 50%-55%, 55%-65%, 65%-75%, 75%-85%, 85%-95%, or 95%-100%), e.g., as measured by surface plasmon resonance, biolayer interferometry, or an enzyme-linked immunosorbent assay (ELISA).

In some aspects, the modulator causes a decrease in infection of a cell by HCMV relative to infection in the absence of the modulator. In some aspects, infection is decreased by at least 40%, as measured in a viral infection assay or a viral entry assay using pseudotyped particles. In some aspects, the decrease is at least 5%, 10%, 15%, 20%, 25%, 30%, 35%, 40%, 45%, 50%, 55%, 60%, 65%, 70%, 75%, 80%, 85%, 90%, 95%, 98%, or 99% or is 100% (i.e., no infection occurs), e.g., the decrease is 5%-15%, 15%-25%, 25%-35%, 35%-45%, 45%-55%, 55%-65%, 65%-75%, 75%-85%, 85%-95%, or 95%-100%).

### C. Modulators of the interaction between B2M and the HCMV gH/gL/UL128-131A pentamer

In some aspects, the disclosure features a modulator of the interaction between the human cytomegalovirus (HCMV) gH/gL/UL128-131A pentamer and beta-2-microglobulin (B2M) that causes a decrease in the binding of the gH/gL/UL128-131A pentamer to B2M.

In some aspects, the modulator decreases binding of the gH/gL/UL128-131A pentamer to B2M by at least 50%. In some aspects, the decrease in binding is at least 5%, 10%, 15%, 20%, 25%, 30%, 35%, 40%, 45%, 50%, 55%, 60%, 65%, 70%, 75%, 80%, 85%, 90%, 95%, 98%, or 99% or is 100% (i.e., binding is abolished), e.g., the decrease is 5%-15%, 15%-25%, 25%-35%, 35%-45%, 45%-55%, 55%-65%, 65%-75%, 75%-85%, 85%-95%, or 95%-100%, relative to binding in the absence of the modulator. In some aspects, the modulator decreases binding of the gH/gL/UL128-131A pentamer to B2M by at least 90% (e.g., 90%-100%). In some aspects, the decrease in binding is at least 50% (e.g., is 50%-55%, 55%-65%, 65%-75%, 75%-85%, 85%-95%, or 95%-100%), e.g., as measured by surface plasmon resonance, biolayer interferometry, or an enzyme-linked immunosorbent assay (ELISA).

In some aspects, the modulator causes a decrease in infection of a cell by HCMV relative to infection in the absence of the modulator. In some aspects, infection is decreased by at least 40%, as measured in a viral infection assay or a viral entry assay using pseudotyped particles. In some aspects, the decrease is at least 5%, 10%, 15%, 20%, 25%, 30%, 35%, 40%, 45%, 50%, 55%, 60%, 65%, 70%, 75%, 80%, 85%, 90%, 95%, 98%, or 99% or is 100% (i.e., no infection occurs), e.g., the decrease is 5%-15%, 15%-25%, 25%-35%, 35%-45%, 45%-55%, 55%-65%, 65%-75%, 75%-85%, 85%-95%, or 95%-100%).

### D. Small molecules

In some aspects, the modulator or candidate modulator is a small molecule. Small molecules are molecules other than binding polypeptides or antibodies as defined herein. Binding small molecules may be identified and chemically synthesized using known methodology (see, e.g., PCT Publication Nos. WO00/00823 and WO00/39585). Binding small molecules are usually less than about 2000 daltons in size (e.g., less than about 2000, 1500, 750, 500, 250 or 200 daltons in size), wherein such organic small molecules that are capable of binding, preferably specifically, to a polypeptide as described herein may be identified without undue experimentation using well known techniques. In this regard, it is noted that techniques for screening small molecule libraries for molecules that are capable of binding to a polypeptide target are well known in the art (see, e.g., PCT Publication Nos. WO00/00823 and WO00/39585). Binding small molecules may be, for example, aldehydes, ketones, oximes, hydrazones, semicarbazones, carbazides, primary amines, secondary amines, tertiary amines, N-substituted hydrazines, hydrazides, alcohols, ethers, thiols, thioethers, disulfides, carboxylic acids, esters, amides, ureas, carbamates, carbonates, ketals, thioketals, acetals, thioacetals, aryl halides, aryl sulfonates, alkyl halides, alkyl sulfonates, aromatic compounds, heterocyclic compounds, anilines, alkenes, alkynes, diols, amino alcohols, oxazolidines, oxazolines, thiazolidines, thiazolines, enamines, sulfonamides, epoxides, aziridines, isocyanates, sulfonyl chlorides, diazo compounds, acid chlorides, or the like.

In some aspects, the binding of NRP2, THBD, or B2M to the HCMV gH/gL/UL128-131A pentamer is decreased (e.g., decreased by 5%, 10%, 20%, 30%, 40%, 50%, 60%, 70%, 80%, 90%, or 100%, e.g., decreased by 5%-15%, 15%-25%, 25%-35%, 35%-45%, 45%-55%, 55%-65%, 65%-75%, 75%-85%, 85%-95%, or 95%-100%) in the presence of the small molecule. In some aspects, the binding of NRP2, THBD, or B2M to the HCMV gH/gL/UL128-131A pentamer is increased (e.g., increased by 5%, 10%, 20%, 30%, 40%, 50%, 60%, 70%, 80%, 90%, 100%, or more than 100%, e.g., increased by 5%-15%, 15%-25%, 25%-35%, 35%-45%, 45%-55%, 55%-65%, 65%-75%, 75%-85%, 85%-95%, 95%-100%, or more than 100%) in the presence of the small molecule. In some aspects, a downstream activity of NRP2, THBD, B2M, and/or HCMV gH/gL/UL128-131A pentamer (e.g., infection of a cell by HCMV) is decreased (e.g., decreased by 5%, 10%, 20%, 30%, 40%, 50%, 60%, 70%, 80%, 90%, or 100%, e.g., decreased by 5%-15%, 15%-25%, 25%-35%, 35%-45%, 45%-55%, 55%-65%, 65%-75%, 75%-85%, 85%-95%, or 95%-100%) in the presence of the small molecule.

### E. Antibodies and antigen-binding fragments

In some aspects, the modulator or candidate modulator is an antibody or an antigen-binding fragment thereof binding NRP2, THBD, B2M, and/or HCMV gH/gL/UL128-131A pentamer. In some aspects, the antigen-binding fragment is a bis-Fab, an Fv, a Fab, a Fab'-SH, a F(ab')$_2$, a diabody, a linear antibody, an scFv, an ScFab, a VH domain, or a VHH domain.

In some aspects, the modulator is a multispecific antibody, e.g., a bispecific antibody. In some aspects, the modulator is a bispecific or multispecific antibody that binds multiple epitopes of NRP2, THBD, B2M, and/or HCMV gH/gL/UL 128-131A pentamer. In some aspects, the modulator is a bispecific or multispecific antibody that binds two or more of NRP2, THBD, B2M, and the HCMV gH/gL/UL128-131A pentamer.

In some aspects, the binding of NRP2, THBD, or B2M to the HCMV gH/gL/UL128-131A pentamer is decreased (e.g., decreased by 5%, 10%, 20%, 30%, 40%, 50%, 60%, 70%, 80%, 90%, or 100%, e.g., decreased by 5%-15%, 15%-25%, 25%-35%, 35%-45%, 45%-55%, 55%-65%, 65%-75%, 75%-85%, 85%-95%, or 95%-100%) in the presence of the antibody or antigen-binding fragment. In some aspects, the binding of NRP2, THBD, or B2M to the HCMV gH/gL/UL 128-131A pentamer is increased (e.g., increased by 5%, 10%, 20%, 30%, 40%, 50%, 60%, 70%, 80%, 90%, 100%, or more than 100%, e.g., increased by 5%-15%, 15%-25%, 25%-35%, 35%-45%, 45%-55%, 55%-65%, 65%-75%, 75%-85%, 85%-95%, 95%-100%, or more than 100%) in the presence of the antibody or antigen-binding fragment. In some aspects, a downstream activity of NRP2, THBD, B2M, and/or the HCMV gH/gL/UL 128-131A pentamer (e.g., infection of a cell by HCMV) is decreased (e.g., decreased by 5%, 10%, 20%, 30%, 40%, 50%, 60%, 70%, 80%, 90%, or 100%, e.g., decreased by 5%-15%, 15%-25%, 25%-35%, 35%-45%, 45%-55%, 55%-65%, 65%-75%, 75%-85%, 85%-95%, or 95%-100%) in the presence of the antibody or antigen-binding fragment.

### F. Peptides

In some aspects, the modulator or candidate modulator is a peptide that binds to NRP2, THBD, B2M, and/or the HCMV gH/gL/UL128-131A pentamer. The peptide may be the peptide may be naturally occurring or may be engineered. The peptide may bind the binding partner with equal, less, or greater affinity than the full-length protein. In some aspects, the peptide performs all functions of the full-length protein. In other aspects, the peptide does not perform all functions of the full-length protein.

In some aspects, the binding of NRP2, THBD, or B2M to the HCMV gH/gL/UL128-131A pentamer is decreased (e.g., decreased by 5%, 10%, 20%, 30%, 40%, 50%, 60%, 70%, 80%, 90%, or 100%, e.g., decreased by 5%-15%, 15%-25%, 25%-35%, 35%-45%, 45%-55%, 55%-65%, 65%-75%, 75%-85%, 85%-95%, or 95%-100%) in the presence of the peptide. In some aspects, the binding of NRP2, THBD, or B2M to the HCMV gH/gL/UL128-131A pentamer is increased (e.g., increased by 5%, 10%, 20%, 30%, 40%, 50%, 60%, 70%, 80%, 90%, 100%, or more than 100%, e.g., increased by 5%-15%, 15%-25%, 25%-35%, 35%-45%, 45%-55%, 55%-65%, 65%-75%, 75%-85%, 85%-95%, 95%-100%, or more than 100%) in the presence of the peptide. In some aspects, a downstream activity NRP2, THBD, B2M, and/or HCMV gH/gL/UL128-131A pentamer (e.g., infection of a cell by HCMV) is decreased (e.g., decreased by 5%, 10%, 20%, 30%, 40%, 50%, 60%, 70%, 80%, 90%, or 100%, e.g., decreased by 5%-15%, 15%-25%, 25%-35%, 35%-45%, 45%-55%, 55%-65%, 65%-75%, 75%-85%, 85%-95%, or 95%-100%) in the presence of the peptide.

### G. Mimics

In some aspects, the modulator or candidate modulator is a mimic, e.g., a molecular mimic, that binds to NRP2, THBD, B2M, and/or the HCMV gH/gL/UL128-131A pentamer. In some aspects, the mimic may perform all functions of the mimicked polypeptide. In other aspects, the mimic does not perform all functions of the mimicked polypeptide.

In some aspects, the binding of NRP2, THBD, or B2M to the HCMV gH/gL/UL128-131A pentamer is decreased (e.g., decreased by 5%, 10%, 20%, 30%, 40%, 50%, 60%, 70%, 80%, 90%, or 100%, e.g., decreased by 5%-15%, 15%-25%, 25%-35%, 35%-45%, 45%-55%, 55%-65%, 65%-75%, 75%-85%, 85%-95%, or 95%-100%) in the presence of the mimic. In some aspects, the binding of NRP2, THBD, or B2M to the HCMV gH/gL/UL128-131A pentamer is increased (e.g., increased by 5%, 10%, 20%, 30%, 40%, 50%, 60%, 70%, 80%, 90%, 100%, or more than 100%, e.g., increased by 5%-15%, 15%-25%, 25%-35%, 35%-45%, 45%-55%, 55%-65%, 65%-75%, 75%-85%, 85%-95%, 95%-100%, or more than 100%) in the presence of the mimic. In some aspects, a downstream activity of NRP2, THBD, B2M, and/or the HCMV gH/gL/UL 128-131A pentamer (e.g., infection of a cell by HCMV) is decreased (e.g., decreased by 5%, 10%, 20%, 30%, 40%, 50%, 60%, 70%, 80%, 90%, or 100%, e.g., decreased by 5%-15%, 15%-25%, 25%-35%, 35%-45%, 45%-55%, 55%-65%, 65%-75%, 75%-85%, 85%-95%, or 95%-100%) in the presence of the mimic.

### H. Assays for modulation of protein-protein interactions

In some aspects, the binding of NRP2, THBD, or B2M to the HCMV gH/gL/UL128-131A pentamer in the presence or absence of the candidate modulator is assessed in an assay for protein-protein interaction. Modulation of the interaction may be identified as an increase in protein-protein interaction in the presence of the modulator compared to protein-protein interaction in the absence of the modulator, e.g., an increase of 5%, 10%, 15%, 20%, 25%, 30%, 35%, 40%, 45%, 50%, 55%, 60%, 65%, 70%, 80%, 90%, 95%, 100%, or more than 100% (e.g., 5%-15%, 15%-25%, 25%-35%, 35%-45%, 45%-55%, 55%-65%, 65%-75%, 75%-85%, 85%-95%, 95%-100%, or more than 100%) in protein-protein interaction. Alternatively, modulation may be identified as a decrease in protein-protein interaction in the presence of the modulator compared to protein-protein interaction in the absence of the modulator, e.g., a decrease of 5%, 10%, 15%, 20%, 25%, 30%, 35%, 40%, 45%, 50%, 55%, 60%, 65%, 70%, 80%, 90%, 95%, or 100% (e.g., 5%-15%, 15%-25%, 25%-35%, 35%-45%, 45%-55%, 55%-65%, 65%-75%, 75%-85%, 85%-95%, or 95%-100%) in protein-protein interaction. The assay for protein-protein interaction may be, e.g., an SPR assay, a biolayer interferometry (BLI) assay, an enzyme-linked immunosorbent assay (ELISA), an extracellular interaction assay, or a cell surface interaction assay.

Exemplary methods for identifying modulators of protein-protein interactions, as well as agents that may modulate such interactions, are described in PCT/US2020/025471, which is hereby incorporated by reference in its entirety.

## III. METHODS OF TREATING OR PREVENTING HCMV INFECTIONS

### A. Methods of treating individuals having HCMV infections

In some aspects, the disclosure features a method for treating an HCMV infection in an individual, the method comprising administering to the individual an effective amount of a modulator described herein (e.g., a modulator of the interaction between NRP2, THBD, and/or B2M and the HCMV gH/gL/UL128-131A pentamer), thereby treating the individual. In some aspects, the disclosure features use of a modulator described herein (e.g., a modulator of the interaction between the NRP2, THBD, and/or B2M and the HCMV gH/gL/UL 128-131A pentamer) in the manufacture of a medicament for treating an HCMV infection in the individual. In some aspects, the individual is immunocompromised, is pregnant, or is an infant.

In some aspects, the duration or severity of HCMV infection is decreased by at least 40% (e.g., decreased by 40%-45%, 45%-55%, 55%-65%, 65%-75%, 75%-85%, 85%-95%, or 95%-100%) relative to an individual who has not been administered the modulator. In some aspects, the duration or severity of HCMV infection is decreased by at least 5%, 10%, 15%, 20%, 25%, 30%, 35%, 40%, 45%, 50%, 55%, 60%, 65%, 70%, 80%, 90%, 95%, or 100% (e.g., 5%-15%, 15%-25%, 25%-35%, 35%-45%, 45%-55%, 55%-65%, 65%-75%, 75%-85%, 85%-95%, or 95%-100%).

### B. Methods of preventing HCMV infection or secondary infection

In some aspects, the disclosure features a method for preventing an HCMV infection in an individual, the method comprising administering to the individual an effective amount of a modulator described herein (e.g., a modulator of the interaction between the NRP2, THBD, and/or B2M and the HCMV gH/gL/UL128-131A pentamer), thereby preventing an HCMV infection in the individual. In some aspects, the disclosure features use of a modulator described herein (e.g., a modulator of the interaction between the NRP2, THBD, and/or B2M and the HCMV gH/gL/UL128-131A pentamer) in the manufacture of a medicament for preventing an HCMV infection in the individual.

In some aspects, the modulator decreases the likelihood of a HCMV infection in the individual relative to infection in the absence of the modulator. In some aspects, the likelihood, extent, or severity of HCMV infection is decreased in patients treated according to the above-described methods relative to untreated patients or relative to patients treated using a control method (e.g., SOC), e.g., decreased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 99% (e.g., decreased by 5%-15%, 15%-25%, 25%-35%, 35%-45%, 45%-55%, 55%-65%, 65%-75%, 75%-85%, 85%-95%, or 95%-100%).

In some aspects, the disclosure features a method of prophylaxis against a secondary HCMV infection in an individual (e.g., an individual having an HCMV infection), the method comprising administering to the individual an effective amount of a modulator described herein (e.g., a modulator of the interaction between NRP2, THBD, and/or B2M and the HCMV gH/gL/UL128-131A pentamer), thereby preventing a secondary HCMV infection in the individual. In some aspects, the disclosure features use of a modulator described herein (e.g., a modulator of the interaction between the NRP2, THBD, and/or B2M and the HCMV gH/gL/UL128-131A pentamer) in the manufacture of a medicament for prophylaxis against a secondary HCMV infection in an individual. In some aspects, the secondary infection is an infection by HCMV of an uninfected tissue.

In some aspects, the modulator decreases the likelihood of a secondary HCMV infection in the individual relative to secondary infection in the absence of the modulator. In some aspects, the likelihood, extent, or severity of secondary HCMV infection is decreased in patients treated according to the above-described methods relative to untreated patients or relative to patients treated using a control method (e.g., SOC), e.g., decreased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 99% (e.g., decreased by 5%-15%, 15%-25%, 25%-35%, 35%-45%, 45%-55%, 55%-65%, 65%-75%, 75%-85%, 85%-95%, or 95%-100%).

*C. Combination therapies*

In some aspects of the above-described methods of treatment and prophylaxis, the method comprises administering to the individual at least one additional therapy (e.g., one, two, three, four, or more than four additional therapies). The modulator of the interaction between NRP2, THBD, and/or B2M and the HCMV gH/gL/UL128-131A pentamer may be administered to the individual prior to, concurrently with, or after the at least one additional therapy.

In some aspects, the additional therapy is a modulator of an interaction between a host cell protein (e.g., a plasma membrane-expressed host cell protein) and the HCMV gHgLgO trimer, e.g., a modulator that decreases the binding of the HCMV gHgLgO trimer to the host cell protein. Exemplary modulators of such interactions are provided in PCT/US2021/060887, which is incorporated by reference herein in its entirety.

## D. Methods of delivery

The compositions utilized in the methods described herein (e.g., a modulator of an interaction between NRP2, THBD, and/or B2M and the HCMV gH/gL/UL128-131A pentamer, e.g., a small molecule, an antibody, an antigen-binding fragment, a peptide, a mimic, an antisense oligonucleotide, or an siRNA) can be administered by any suitable method, including, for example, intravenously, intramuscularly, subcutaneously, intradermally, percutaneously, intraarterially, intraperitoneally, intralesionally, intracranially, intraarticularly, intraprostatically, intrapleurally, intratracheally, intrathecally, intranasally, intravaginally, intrarectally, topically, intratumorally, peritoneally, subconjunctivally, intravesicularly, mucosally, intrapericardially, intraumbilically, intraocularly, intraorbitally, orally, transdermally, intravitreally (e.g., by intravitreal injection), by eye drop, by inhalation, by injection, by implantation, by infusion, by continuous infusion, by localized perfusion bathing target cells directly, by catheter, by lavage, in cremes, or in lipid compositions. The compositions utilized in the methods described herein can also be administered systemically or locally. The method of administration can vary depending on various factors (e.g., the compound or composition being administered and the severity of the condition, disease, or disorder being treated). In some aspects, a modulator of a protein-protein interaction is administered intravenously, intramuscularly, subcutaneously, topically, orally, transdermally, intraperitoneally, intraorbitally, by implantation, by inhalation, intrathecally, intraventricularly, or intranasally. Dosing can be by any suitable route, e.g., by injections, such as intravenous or subcutaneous injections, depending in part on whether the administration is brief or chronic. Various dosing schedules including but not limited to single or multiple administrations over various time-points, bolus administration, and pulse infusion are contemplated herein.

A modulator of a protein-protein interaction described herein (and any additional therapeutic agent) may be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The modulator need not be, but is optionally formulated with and/or administered concurrently with one or more agents currently used to prevent or treat the disorder in question. The effective amount of such other agents depends on the amount of the modulator present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

## III. METHODS OF IDENTIFYING A MODULATOR OF THE INTERACTION BETWEEN the HCMV gH/gL/ UL128-131A pentamer and B2M

### A. Assays for modulation of interaction

In some aspects, the invention features identifying a modulator of the interaction between the HCMV gH/gL/UL128-131A pentamer and beta-2-microglobulin (B2M), the method comprising: (a) providing a candidate modulator (e.g., a candidate modulator described in Section II herein); (b) contacting the HCMV gH/gL/UL128-131A pentamer with B2M in the presence or absence of the candidate modulator under conditions permitting the binding of the HCMV gH/gL/UL128-131A pentamer to B2M; and (c) measuring the binding of the HCMV gH/gL/UL128-131A pentamer to B2M, wherein an increase or decrease in binding in the presence of the candidate modulator relative to binding in the absence of the candidate modulator identifies the candidate modulator as a modulator of the interaction between the HCMV gH/gL/UL128-131A pentamer and B2M.

In some aspects, the disclosure features a method of identifying a modulator of a downstream activity of the HCMV gH/gL/UL128-131A pentamer, the method comprising (a) providing a candidate modulator; (b) contacting the HCMV gH/gL/UL128-131A pentamer with B2M in the presence or absence of the candidate modulator under conditions permitting the binding of the HCMV gH/gL/UL128-131A pentamer to B2M; and (c) measuring a downstream activity of the HCMV gH/gL/UL128-131A pentamer, wherein a change in the downstream activity in the presence of the candidate modulator relative to the downstream activity in the absence of the candidate modulator identifies the candidate modulator as a modulator of the downstream activity of the HCMV gH/gL/UL128-131A pentamer.

In some aspects, the disclosure features a method of identifying a modulator of a downstream activity of B2M, the method comprising (a) providing a candidate modulator; (b) contacting B2M with the HCMV gH/gL/UL128-131A pentamer in the presence or absence of the candidate modulator under conditions permitting the binding of B2M to the HCMV gH/gL/UL 128-131A pentamer; and (c) measuring a downstream activity of B2M, wherein a change in the downstream activity in the presence of the candidate modulator relative to the downstream activity in the absence of the candidate modulator identifies the candidate modulator as a modulator of the downstream activity of B2M.

In some aspects, the increase or decrease in binding is at least 50% (e.g., 50%-55%, 55%-65%, 65%-75%, 75%-85%, 85%-95%, or 95%-100%), as measured by surface plasmon resonance, biolayer interferometry, or an enzyme-linked immunosorbent assay (ELISA).

In some aspects, the modulator is an inhibitor of the downstream activity of the HCMV gH/gL/UL 128-131A pentamer or B2M.

In some aspects, the modulator is a modulator as described in Section II herein, e.g., is a small molecule, an antibody or antigen-binding fragment thereof (e.g., a bis-Fab, an Fv, a Fab, a Fab'-SH, a F(ab')$_2$, a diabody, a linear antibody, an scFv, an scFab, a VH domain, or a VHH domain), a peptide, a mimic, or an inhibitory nucleic acid (e.g., an ASO or a siRNA).

In some aspects in which the modulator is an antibody or antigen-binding fragment thereof, the antibody or antigen-binding fragment thereof binds the HCMV gH/gL/UL128-131A pentamer. In some aspects, the antibody or antigen-binding fragment thereof binds B2M. For example, in some aspects, the modulator is an antibody or antigen-binding fragment thereof that binds the HCMV gH/gL/UL128-131A pentamer, an antibody or antigen-binding fragment thereof that binds B2M, or an antibody or antigen-binding fragment thereof that binds the HCMV gH/gL/UL128-131A pentamer and B2M.

In some aspects, the change in the downstream activity is a decrease in the amount, strength, or duration of the downstream activity.

In some aspects, the downstream activity is infection of a cell by HCMV, and infection is decreased in the presence of the modulator. The decrease in infection may be a decrease of at least 5%, at least 10%, at least 20%, at least 30%, at least 40%, at least 50%, at least 60%, at least 70%, at least 80%, at least 90%, or 100% (e.g., may be a decrease of 5%-15%, 15%-25%, 25%-35%, 35%-45%, 45%-55%, 55%-65%, 65%-75%, 75%-85%, 85%-95%, or 95%-100%), e.g., as measured in a viral infection assay or a viral entry assay using pseudotyped particles. In some aspects, infection is decreased by at least 40% as measured in a viral infection assay or a viral entry assay using pseudotyped particles.

In some aspects, the candidate modulator is provided to a cell (e.g., a mammalian cell), to cell culture media, to conditioned media, and/or to a purified form of the HCMV gH/gL/UL128-131A pentamer and/or B2M. In some aspects, the candidate modulator is provided at a concentration of at least 0.1 nM, 0.5 nM, 1 nM, 10 nM, 50 nM, 100 nM, 250 nM, 500 nM, 750 nM, 1 μM, 2 μM, 3 μM, 5 μM, or 10 μM. In some aspects, the candidate modulator is provided at a concentration of between 0.1 nM and 10 μM. In some aspects, the candidate modulator is provided in a solution, e.g., in a soluble form.

In some aspects, the candidate modulator is identified as a modulator if the increase in binding is at least 70%. In some aspects, the increase in binding is at least 5%, at least 10%, at least 20%, at least 30%, at least 40%, at least 50%, at least 60%, at least 70%, at least 80%, at least 90%, at least 100%, or more than 100% (e.g., the increase is 5%-15%, 15%-25%, 25%-35%, 35%-45%, 45%-55%, 55%-65%, 65%-75%, 75%-85%, 85%-95%, 95%-100%, or more than 100%). In some aspects, the increase in binding is at least 70%.

In some aspects, the candidate modulator is identified as a modulator if the decrease in binding is at least 70%. In some aspects, the decrease in binding is at least 5%, at least 10%, at least 20%, at least 30%, at least 40%, at least 50%, at least 60%, at least 70%, at least 80%, at least 90%, or 100% (e.g., the decrease in binding is 5%-15%, 15%-25%, 25%-35%, 35%-45%, 45%-55%, 55%-65%, 65%-75%, 75%-85%, 85%-95%, or 95%-100%). In some aspects, the decrease in binding is at least 70%.

*i. Assays for modulation of protein-protein interaction*

In some aspects, the binding of the HCMV gH/gL/UL128-131A pentamer and B2M in the presence or absence of the candidate modulator is assessed in an assay for protein-protein interaction. Modulation of the interaction between the HCMV gH/gL/UL 128-131A pentamer and B2M may be identified as an increase in protein-protein interaction in the presence of the modulator compared to protein-protein interaction in the absence of the modulator, e.g., an increase of 5%, 10%, 15%, 20%, 25%, 30%, 35%, 40%, 45%, 50%, 55%, 60%, 65%, 70%, 80%, 90%, 95%, 100%, or more than 100% (e.g., an increase of 5%-15%, 15%-25%, 25%-35%, 35%-45%, 45%-55%, 55%-65%, 65%-75%, 75%-85%, 85%-95%, 95%-100%, or more than 100%) in protein-protein interaction. Alternatively, modulation may be identified as a decrease in protein-protein interaction in the presence of the modulator compared to protein-protein interaction in the absence of the modulator, e.g., a decrease of 5%, 10%, 15%, 20%, 25%, 30%, 35%, 40%, 45%, 50%, 55%, 60%, 65%, 70%, 80%, 90%, 95%, or 100% (e.g., a decrease of 5%-15%, 15%-25%, 25%-35%, 35%-45%, 45%-55%, 55%-65%, 65%-75%, 75%-85%, 85%-95%, or 95%-100%) in protein-protein interaction. The assay for protein-protein interaction may be, e.g., an SPR assay, a biolayer interferometry (BLI) assay, an enzyme-linked immunosorbent assay (ELISA), an extracellular interaction assay as described in WO 2020/205626, or a cell surface interaction assay as described in WO 2020/205626.

All patent, patent publication and literature references cited in the present specification are hereby incorporated by reference in their entirety.

## V. EXAMPLES

### Example 1. Structural basis for HCMV pentamer receptor recognition and antibody neutralization *A. Introduction*

Human cytomegalovirus (HCMV) is a member of the Betaherpesvirus family, responsible for severe morbidity and mortality in immunocompromised individuals (Kotton et al., Nat. Rev. Nephrol., 6: 711-721, 2010). HCMV infection is particularly threatening during pregnancy, and represents the leading viral cause of congenital birth defects (Hyde et al., Reviews in Medical Virology, 20(5): 311-326, 2010).

HCMV exhibits a broad cellular tropism and utilizes the gH/gL/UL128-130-131A pentamer complex ("pentamer") to bind to different receptor proteins and infect diverse cell types including epithelial, endothelial and myeloid cells (Connolly et al., Nat. Rev. Microbiol., 19(2), 110-121, 2021; E et al., Proc. Natl. Acad. Sci., 116(14): 7043-7052, 2019; Martinez-Martin et al., Cell, 174(5): 1158-1171, 2018; Nguyen and Kamil, Viruses, 10(12): 704, 2018). HCMV receptor binding is thought to provide the triggering signal for the gB glycoprotein to catalyze membrane fusion between virus and host cells, allowing HCMV to enter cells, replicate and establish its latency (Malito et al., Curr. Opin. Virol., 31: 43-51, 2018). Neuropilin2 (NRP2) deficiency has been demonstrated to make select endothelial and epithelial cells resistant to HCMV infection, indicating a key role of NRP2 as a HCMV receptor (Martinez-Martin et al., Cell, 174(5): 1158-1171, 2018), but the structural basis for this interaction has remained unknown. The HCMV pentamer also binds to thrombomodulin (THBD) with nanomolar affinity, but a role for THBD in the entry of HCMV has remained enigmatic (Martinez-Martin et al., Cell, 174(5): 1158-1171, 2018).

Upon infection, HCMV elicits extremely potent neutralizing antibodies against the pentamer, underlining the importance of this complex as a therapeutic and vaccine candidate against HCMV (Macagno et al., J. Virol., 84(2): 1005-1013, 2010). Despite considerable efforts, a vaccine against HCMV still represents an unmet medical need, and no approved therapy is currently available to treat congenital HCMV infection (Biron, Antiviral Research, 71(2-3): 154-163, 2006; Chen et al., Viruses, 12(1): 21, 2019). This gap is largely due to the lack of a structural understanding of the interaction between the pentamer and host cellular receptors, and limited knowledge of the epitopes relevant for pentamer neutralization.

In the present Examples, high-resolution structures of the pentamer in complex with the cellular receptors NRP2 and THBD are determined. These structures highlight the specific interactions required for HCMV pentamer-receptor recognition and reveal an unanticipated dimerization of the pentamer-receptor complexes. Importantly, it was found that the interactions between the pentamer and NRP2 or THBD are mutually exclusive, and that both are functional receptors mediating entry into different cell types, rather than being co-receptors. The present Examples also report the structures of the pentamer bound to a set of potent neutralizing antibodies that identify key neutralizing sites against HCMV. These studies provide a framework for understanding HCMV receptor recognition and antibody-based neutralization.

### *B. Summary of results*

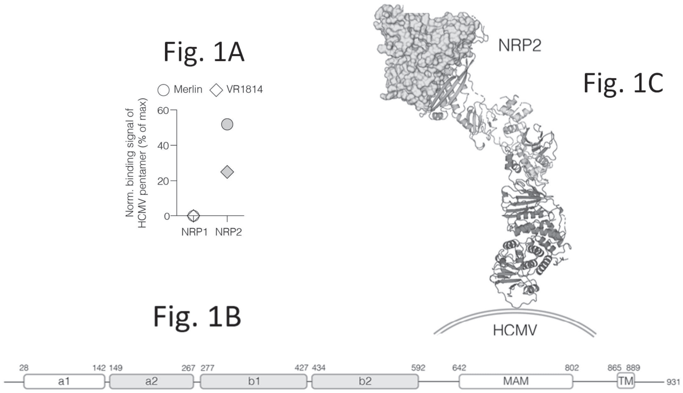

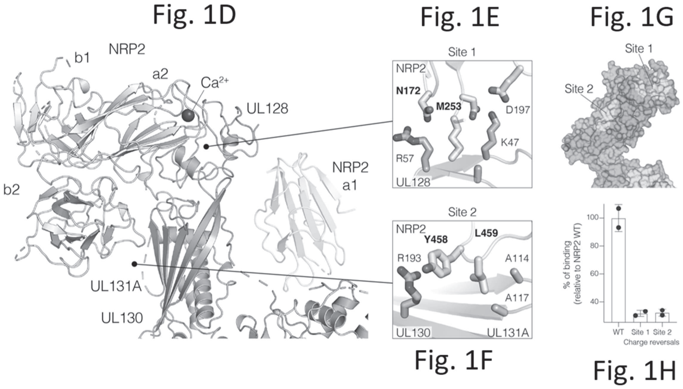

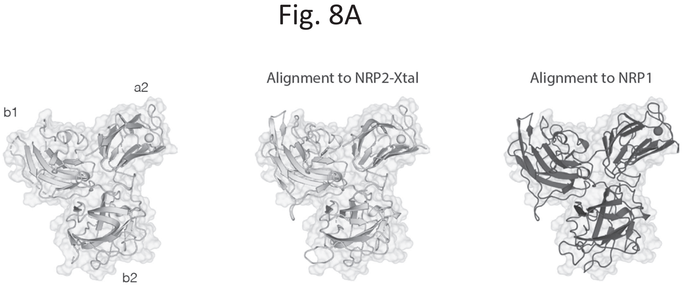

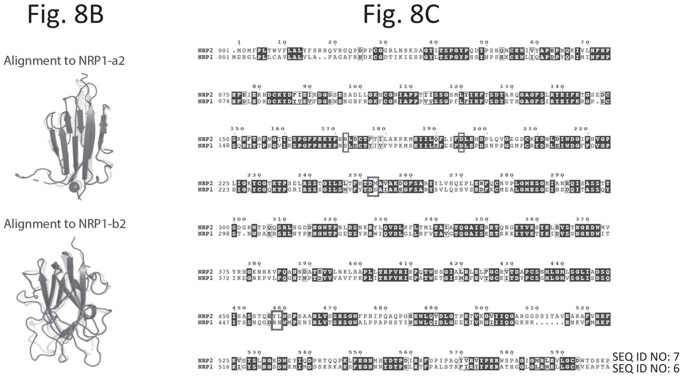

The Examples provided herein present a comprehensive structural, biophysical, and functional analysis of the HCMV pentamer to provide critical insight into its architecture, receptor recognition, cell entry, and neutralization. These studies reveal how a large portion of subunits UL128-131A of the pentamer binds to two completely different functional receptor proteins, NRP2 and THBD, which sheds light on how HCMV achieves broad cellular tropism and receptor specificity of different cell types. Neuropilins 1 and 2 have been shown to use their b1 domain to interact with the CendR motif of binding partners, including VEGF the and spike protein of SARS-CoV-2 (Daly et al., Science, 370(6518): 861-865, 2020; Parker et al., J. Biol. Chem., 287(14): 11082-11089, 2012); however, structural studies now demonstrate that NRP2 does not utilize this canonical binding site, but instead engages the pentamer through key interactions between the a2 and b2 domains to the UL128 and UL131A subunits (Figs. 1E, 1F, **1H,** and 8A; Wrapp et al., bioRxiv, p. 2021.03.25.436804, 2021). Upon pentamer binding, the NRP2 a1 domain is displaced from the a2b1b2 core and repositioned at the site of attachment between UL128 and gL (Fig. 1D). Notably, the NRP2 a1 domain binds to the same region of pentamer recognized by the THBD receptor (Figs. 1A-1H and 2A-2D), indicating that HCMV pentamer has evolved an opportunistic binding site able to recognize multiple distinct interacting partners with high affinity to enter different cell types.

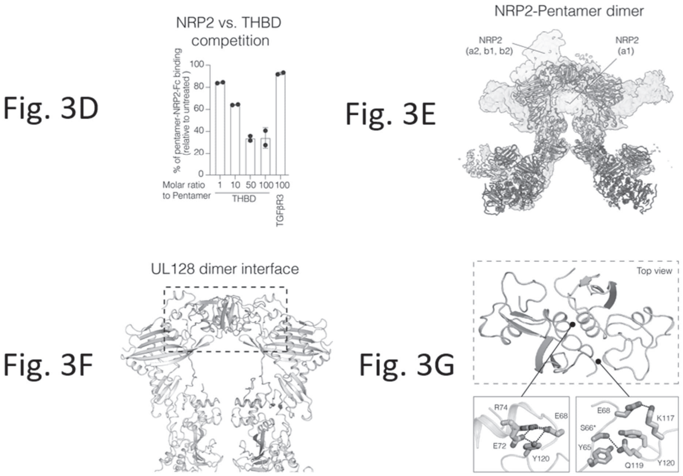

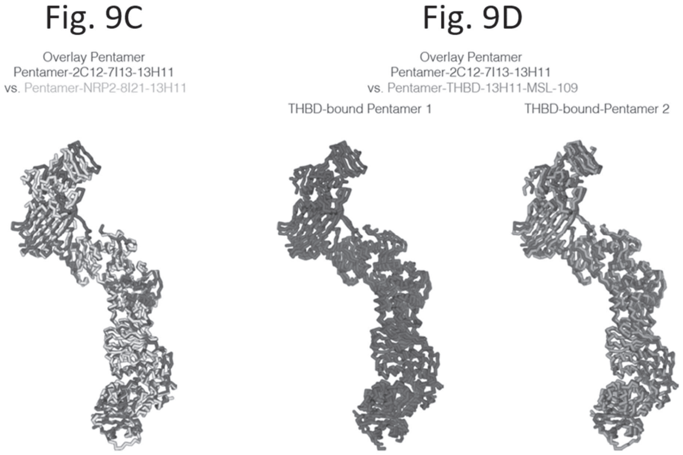

In these Examples, it is shown for the first time that THBD is a relevant functional receptor for HCMV (Figs. 3A and 3B). The mutually exclusive binding of NRP2 and THBD to the pentamer revealed by these structural and biophysical studies (Figs. 3D-3F) further suggests that HCMV likely uses the pentamer to enter epithelial and endothelial cells through two alternative pathways, rather than using these proteins as co-receptors. Based on the expression profiles of NRP2 and THBD, it is possible that the NRP2 receptor is principally used for epithelial cell infection, while THBD is the main receptor for endothelial and myeloid cell infection (Sartain et al., J. Immunol., 196(2): 832-845, 2016; Bachem et al., J. Exp. Med., 207(6): 1273-1281, 2010). It was previously proposed that the pentamer would undergo a large conformational change upon receptor binding to trigger gB mediated membrane fusion and HCMV entry (Chandramouli et al., Sci. Immunol., 2(30): 2017). However, the present structural studies suggest that NRP2 or THBD interactions do not significantly alter the conformation of the pentamer (Figs. 9C and 9D). Thus, the pentamer might trigger gB activation through a different mechanism without large pentamer conformational changes, perhaps similar to what has been recently proposed for the HCMV trimer (Kschonsak et al., Cell, 184(5): 1232-1244.e16, 2021).

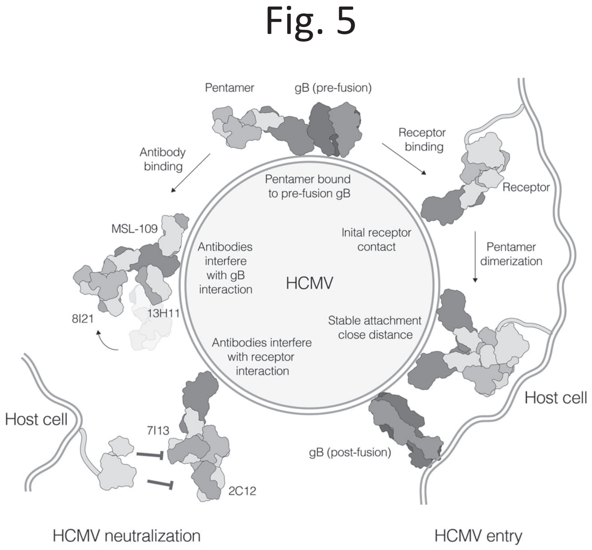

The present structures reveal that the HCMV pentamer can undergo dimerization in which the NRP2 a1 and THBD lectin-like domains become sandwiched in between two pentamers. This apparent receptor-mediated dimerization of the pentamer occurs in a head-to-head fashion through the UL128 subunit and the NRP2 or THBD receptors, respectively (Figs. 2B and 3G). This mode of receptor engagement by the pentamer is distinctly different from what was observed for the HCMV trimer, where each host receptor was seen to engage only one copy of the trimer (Kschonsak et al., Cell, 184(5): 1232-1244.e16, 2021). It is notable that these structural observations appear to be consistent with the experimental designs required for the identification of the human receptors involved in trimer or pentamer recognition (Martinez-Martin et al., Cell, 174(5): 1158-1171, 2018). In fact, whereas PDGFRα was identified as the trimer receptor using a classic affinity purification and mass spectrometry approach (Kabanova et al., Nat. Microbiol., 1(8): 16082, 2016), the identification of physiologically relevant pentamer-receptor interactions could only be enabled through oligomerization of the pentamer, likely via increased avidity for the receptors, and potentially mimicking the molecular arrangement of two opposing membranes (i.e. the viral envelope and host cell) (Martinez-Martin et al., Cell, 174(5): 1158-1171, 2018). The apparent requirement for receptor-mediated dimerization of the pentamer and the lack of structural rearrangements upon receptor binding support the model for pentamer-specific HCMV entry into host cells shown in Fig. 5. In this model, receptor binding is correlated with pentamer dimerization, and in turn mediates receptor clustering to ultimately facilitate a high-affinity and stable tethering of the viral membrane with the host cell membrane. Through a mechanism not yet known, receptor-mediated pentamer-dimerization and membrane tethering would trigger the transition of gB from its pre- to post-fusion conformation and initiate membrane fusion, perhaps through local trapping of gB and/or perturbations within the viral membrane bilayer upon pentamer clustering. The recent high-resolution structure of HCMV gB in its prefusion conformation (Liu et al., Sci. Adv., 7(10), 2021) should open new avenues to test these and other hypotheses. Future high-resolution structural studies will be required to address the structural basis of the HCMV pentamer (or trimer) interaction with prefusion gB and the mechanism required to trigger membrane fusion.

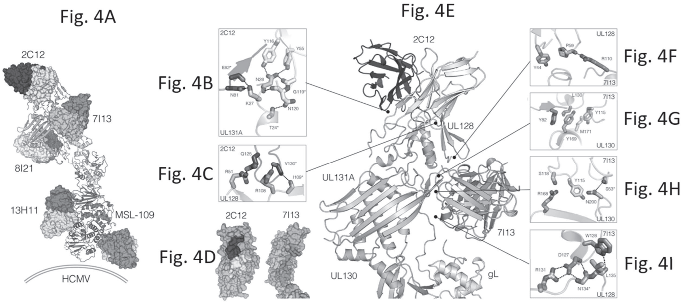

The HCMV pentamer elicits the most potent neutralizing antibody response against HCMV, but the structural basis for this neutralization has remained poorly understood. These results show that Fab 2C12 directly interferes with the NRP2 domain binding and blocks the attachment between the pentamer and NRP2 (Figs. 4A-4M). More importantly, Fab 7113 binds to a surface of the pentamer that would block both NRP2 and THBD, representing a superior neutralizing antibody to block receptor interaction of the pentamer and potentially pentamer dimerization (Figs. 4A-4M). Overall, the present Examples present the structural basis for pentamer receptor engagement and neutralization, which reveals insights into the broad cellular tropism and cell entry mechanism of HCMV. These findings provide a framework for the future development of effective vaccines and therapeutics against HCMV.

### C. Methods

#### i. Generation of HCMV glycoprotein and Fab constructs

Codon-optimized (for expression in human cells) HCMV gH, gL, UL128, UL130, UL131A and NRP2 genes were synthesized and subcloned into the plasmid expression vector pRK5 (Genentech). The gH gene comprised only the extracellular region (first 716 amino acids) and was fused to a C-terminal Myc-Avi-8xHIS tag. For protein purification purposes, UL130 was fused to a C-terminal double Strep tag.

#### ii. Protein expression and purification

The HCMV pentamer was purified in three steps, as previously described for the HCMV trimer (Kschonsak et al., Cell, 184(5): 1232-1244.e16, 2021). EXPI293F™ cells were transfected with plasmids encoding individual subunits. The expression supernatant corresponding to a 50 L expression was concentrated via tangential flow filtration (TFF) to a volume of 1-2 L, loaded on a 20 mL Ni SEPHAROSE™ Excel resin (Cytiva), washed with 13 column volumes (CV) wash buffer (50 mM TRIS pH 8.0, 300 mM NaCl, 5% glycerol, 20 mM imidazole) and eluted in 5 CV elution buffer (50 mM TRIS pH 8.0, 300 mM NaCl, 5% glycerol, 400 mM imidazole). The eluent was applied to 3 mL STREP-TACTIN® XT high affinity resin (IBA Lifesciences) and bound for 2 hours. The resin was washed with 10 CV Strep-wash buffer (25 mM HEPES pH 7.5, 300 mM NaCl, 5% glycerol) and eluted from the beads in Strep-wash buffer supplemented with 50 mM biotin. The eluate was concentrated with an AMICON® Ultra centrifugal filter device (30 kDa MWCO) and loaded on a SUPERDEX™ 200 10/300 or 16/60 column equilibrated in Pentamer-SEC buffer (25 mM HEPES pH 7.5, 300 mM NaCl, 5% glycerol).

Human Fc-tagged NRP2 proteins were expressed in 30 mL cultures of EXPI293F™ cells, transfected with 30 µg of plasmid DNA, and grown for 7 days. After harvesting the expression media culture supernatants, the Fc-fusion proteins were purified with 0.15 mL TOYOPEARL® AF-r Protein A HC-650F (Tosoh Bioscience, Grove City, OH). Unbound proteins and media components were removed by washing with 10 CV of phosphate buffered saline (PBS, pH 7.4). NRP2-huFc fusions were eluted from the resin using elution buffer (50 mM phosphoric acid, pH 2.9) and immediately neutralized to a pH of ~6.0 by adding 0.05 mL of 20X PBS pH 11.0. The proteins were further polished by size exclusion chromatography (SEC) purification, performed on a ZENIX®-C SEC 300 column (Sepax Technology, Newark, DE), connected to an ULTIMATE™ 3000 HPLC System (Thermo Fisher Scientific, Waltham, MA). The column was equilibrated in 1X PBS, pH 7.4 and the samples were separated by isocratic elution at a flowrate of 1.5 ml/minute.

Human THBD-Flag and NRP2-Flag were purified from a 10 L expression supernatant. The supernatant was incubated with 10 mL M2 agarose Flag resin (Sigma) and incubated for 20 hours at 4°C. The resin was washed with 10 CV FLAG-wash buffer (25 mM HEPES pH 7.5, 200 mM NaCl, 5% glycerol) and eluted with FLAG-wash buffer supplemented with 0.2 mg/ml FLAG peptide. The eluate was concentrated with an AMICON® Ultra centrifugal filter device (30 kDa MWCO) and loaded on a SUPERDEX™ 200 10/60 column equilibrated in SEC buffer (25 mM HEPES pH 7.5, 200 mM NaCl).

The heavy and light chains of the fragment antigen-binding region (Fab) MSL-109 were co-expressed under a phoA promoter in E. coli 34B8 cells (in-house strain) in phosphate limiting media (C.R.A.P.) for 20 hours at 30°C. The pellet from a 1 L expression was resuspended in 70 mL lysis buffer (1x PBS, 25 mM EDTA) supplemented with Roche protease inhibitor tablets and lysed by sonication. The lysate was cleared by centrifugation at 25,000 x g for 1 hour and subsequently passed through a 0.45 µm filter. The cleared lysate was loaded onto a 5 mL HiTrap Protein G HP (Cytiva) column that was equilibrated in lysis buffer. The column was washed with 10-20 CV lysis buffer and eluted in 0.58% (v/v) acetic acid. The pH of the eluate was immediately adjusted by addition of SP-A buffer (20 mM MES pH 5.5) and loaded onto a 5 mL HiTrap SP HP cation exchange chromatography column (Cytiva). The Fab was eluted in a linear 20 CV gradient to SP-B buffer (20 mM MES pH 5.5, 500 mM NaCl). The eluate was concentrated using an AMICON® Ultra centrifugal filter device (10 kDa MWCO) and further purified on a SUPERDEX™ 200 10/300 column equilibrated in Fab-S200 buffer (25 mM Tris pH 7.5, 300 mM NaCl). The purified Fab was concentrated an AMICON® Ultra centrifugal filter device (10 kDa MWCO), frozen in liquid $N_2$, and stored at -80°C. The heavy and light chains of the Fabs 2C12, 7I13, 8I21 and 13H11 were co-expressed under a phoA promoter in E. coli 34B8 cells (in-house strain) in phosphate limiting media (C.R.A.P.) for 20 hours at 30°C. The pellet from a 4 L expression was resuspended in 250 mL lysis buffer (25mM Tris pH7.5, 150mM NaCl, 5mM EDTA, 2mM $NaN_3$) supplemented with Roche protease inhibitor tablets and lysed by sonication. Subsequent purification followed a similar protocol as that described for MSL-109.

Complexes between the pentamer, Fabs and THBD were generated by incubation for at least 1 hour on ice with at least 1.2 molar excess of Fab and receptors and purified by SEC to remove excess Fab/receptors. Pentamer-NRP2-Fab complexes were formed in two steps. First, the pentamer and NRP2 were mixed and purified by SEC, followed by addition of excess of Fabs 8I21 and 13H11 and a second SEC purification.

### iii. CryoEM sample preparation and data acquisition

The HCMV pentamer complexes shown in this study were prepared as previously described for the HCMV trimer (Kschonsak et al., Cell, 184(5): 1232-1244.e16, 2021). For the HCMV pentamer gHgLUL128-UL130-UL131A + NRP2 +8I21 + 13H11 complex, holey carbon grids (ULTRAUFOIL® 25 nM Au R 0.6/1 300 mesh; QUANTIFOIL®) were glow-discharged for 20 seconds using the Solarus plasma cleaner (Gatan). For all other pentamer complexes, holey carbon grids (ULTRAUFOIL® 25 nM Au R 0.6/1 300 mesh; QUANTIFOIL®) were incubated with a thiol-reactive, self-assembling reaction mixture of 4mM monothiolalkane(C11)PEG6-OH (11-mercaptoundecyl) hexaethyleneglycol (SPT-0011P6, SensoPath Technologies, Inc., Bozeman, MT) (Meyerson et al., Sci. Rep., 4: 7084, 2014). Grids were incubated with this self-assembled monolayer (SAM) solution for 24 hours. Prior to grid freezing, grids were removed from the SAM solution and rinsed with EtOH. 3 µL of the sample at a concentration of 0.66-1.8 mg/mL was applied to the grid and blotted single-sided with a Leica EM GP (Leica) using 3.5 second blotting time with 100% humidity and plunge-frozen in liquid ethane cooled by liquid nitrogen. The pentamer-NRP2-8I21-13H11 sample was mildly crosslinked with 0.025% EM-grade glutaraldehyde for 10 minutes at room temperature and quenched with 9 mM Tris pH 7.5 prior to grid application.

Movie stacks for HCMV pentamer gHgL-UL128-UL130-UL131A + 2C12 +7I13 + 13H11 and HCMV pentamer gHgL-UL128-UL130-UL131A + NRP2 +8I21 + 13H11 were collected using SerialEM (Mastronarde, J. Struct. Biol.,

152(1): 36-51, 2005) on a Titan Krios G3i (ThermoFisher Scientific, Waltham, MA) operated at 300 keV with bioquantum energy filter equipped with a K2 Summit direct electron detector camera (Gatan Inc., Pleasanton, CA). Images were recorded at 165,000 × magnification corresponding to 0.824 Å per pixel, using a 20 eV energy slit. Each image stack contains 50 frames recorded every 0.2 seconds for an accumulated dose of ~50 e $Å^{-2}$ and a total exposure time of 10 seconds. Images were recorded with a set defocus range of 0.5 to 1.5 $\mu$m.

Movie stacks for HCMV pentamer gHgL-UL128-UL130-UL131A + THBD +MSL-109 + 13H11 were collected using SerialEM (Mastronarde, J. Struct. Biol., 152(1): 36-51, 2005) on a Titan Krios G3i (ThermoFisher Scientific, Waltham, MA) operated at 300 keV with bioquantum energy filter equipped with a K3 Summit direct electron detector camera (Gatan Inc., Pleasanton, CA). Images were recorded in EFTEM mode at 105,000 × magnification corresponding to 0.838 Å per pixel, using a 20 eV energy slit. Each image stack contains 60 frames recorded every 0.05 seconds for an accumulated dose of ~60 e $Å^{-2}$ and a total exposure time of 3 seconds. Images were recorded with a set defocus range of 0.5 to 1.5 $\mu$m.

### iv. CryoEM data processing

CryoEM data were processed using a combination of the RELION (Scheres, J. Struct. Biol., 180(3): 519-530, 2012), cisTEM (Grant et al., eLife, 7:e35383, 2018), and cryoSparc (Punjani et al., Nat. Methods, 14(3): 290-296, 2017) software packages, similar to previous work on the HCMV trimer (Kschonsak et al., Cell, 184(5): 1232-1244.e16, 2021).

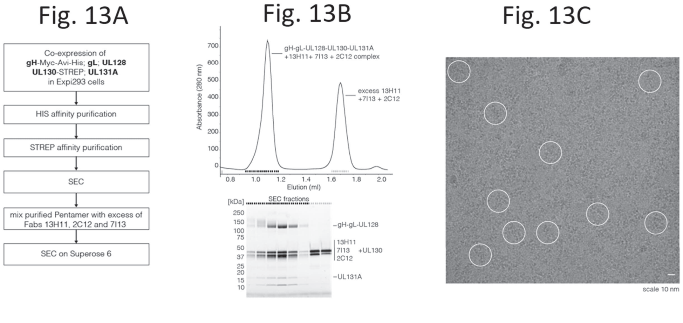

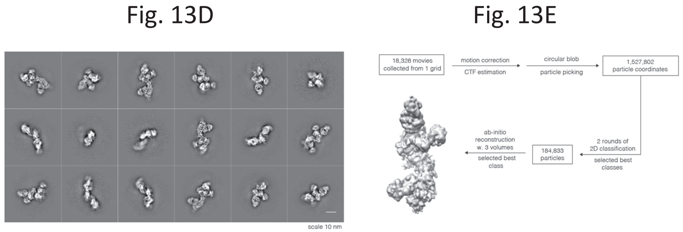

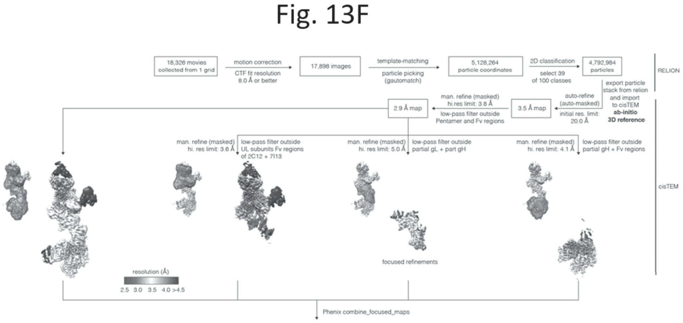

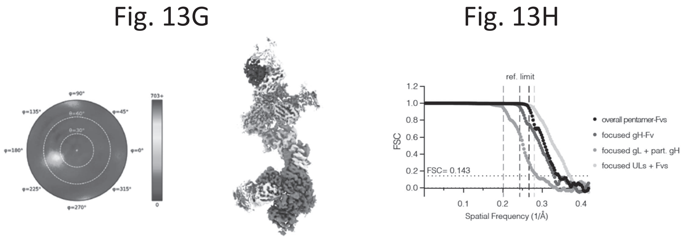

The HCMV pentamer-2C12-7I13-13H11 cryoEM data were processed as described in Figs. 13A-13H. For the generation of the first ab-initio reconstruction, 18,326 movies were motion corrected and contrast-transfer function parameters were fit within cisTEM. A total of 1,527,802,766 potential particles were picked using the circular blob picking tool within cisTEM. Particles were sorted in 2 rounds of cisTEM 2D classification to select the best aligning particles, yielding 184,833 particles. These particles were subjected to an ab-initio 3D generation within cisTEM with three target volumes. The volume corresponding to a HCMV pentamer bound to Fabs 2C12, 7I13 and 13H11 was used as a 3D reference for the high-resolution 3D refinements. For the generation of a high-resolution 3D reconstruction of the pentamer-2C12-7I13-13H11 complex, all 18,326 movies were corrected for frame motion using the MotionCor2 (Zheng et al., Nature Methods, 14: 331-332, 2017) implementation in RELION and contrast-transfer function parameters were fit using the 30-4.5 Å band of the spectrum with CTFFIND-4 (Rohou and Grigorieff, J. Struct. Biol., 192(2): 216-221, 2015). CTF fitted images were filtered on the basis of the detected fit resolution better than 8 Å. A total of 5,128,264 particles were picked by template-matching with gautomatch (MRC Laboratory of Molecular Biology) using a 30 Å low-pass filtered pentamer-2C12-7I13-13H11 complex reference structure. Particles were sorted during RELION 2D classification and 4,792,984 selected particles were imported into cisTEM for 3D refinements. The first pentamer-2C12-7I13-13H11 reconstruction was obtained after auto-refine and manual refinements with a mask around the pentamer-2C12-7I13-13H11 complex and by applying low-pass filter outside the mask (filter resolution 20 Å) and a score threshold of 0.30, so that only the best-scoring 30% of particle images would be included in the 3D reconstruction at each cycle. The weight outside of the mask was incrementally reduced from 0.5 to 0.15 in iterative rounds of manual refinements (no data beyond 3.8 Å were used in the refinement). To improve the quality of the map, focused refinements were conducted after dividing the map into three distinct regions using masks and applying low-pass filter outside the mask (filter resolution 20 Å) and a score threshold of 0.20. The focused maps were sharpened in cisTEM with the following parameters: flattening from a resolution of 8 Å, applying a pre-cut-off B-factor of -90 $Å^2$ from the origin of reciprocal space to 8Å and applying a figure-of-merit filter (Rosenthal and Henderson, J. Mol. Biol., 333(4): 721-745, 2003). For model building and figure preparation, a composite map was generated from the three individual focused 3D maps using phenix combine_focused_maps (Afonine et al., Acta Crystallogr. Sect. D Struct. Biol., 74(6): 531-544, 2018).

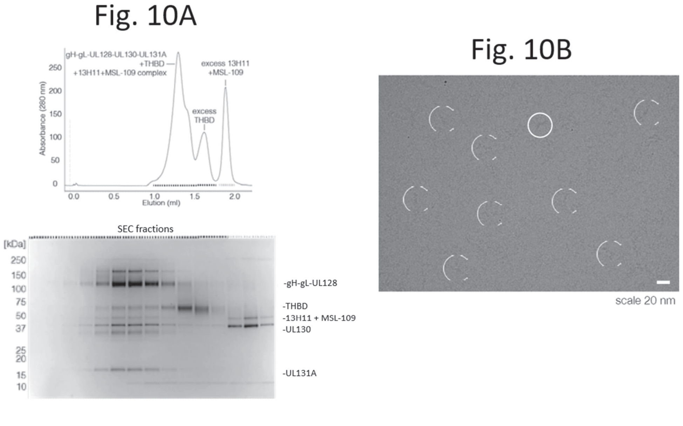

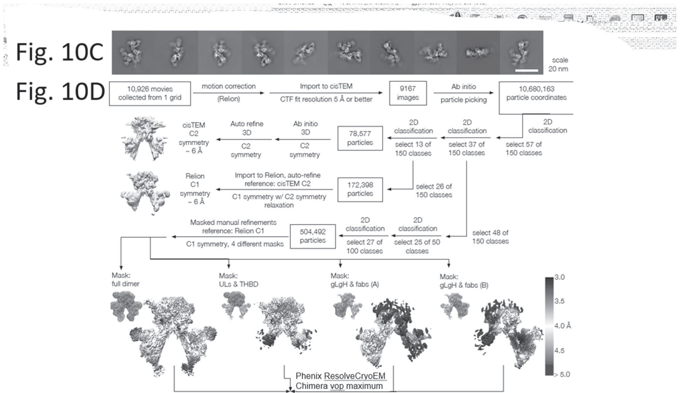

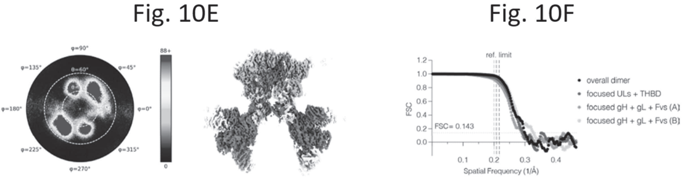

The HCMV pentamer-THBD-13H11-MSL109 cryoEM data were processed as described in Figs. 10A-10F. The 10,926 movies were motion corrected using Relion's implementation of motion correction, and the aligned micrographs were imported into cisTEM for CTF estimation via CTFFIND4. A total of 9,167 images with CTF fit resolutions of at least 5 Å were selected for ab-initio particle picking, using an exclusion and template radius of 30 Å. The resulting 10,680,163 particle coordinates were extracted using a 400 pixel box size, and 1.07 Å per pixel. Multiple rounds of hierarchical 2D classification and class selection ultimately resulted in three particle stacks: A 78,577 particle stack consisting of 2D classes in which an entire dimer was clearly resolved, a 172,398-particle stack containing several additional classes in which the dimer was nearly fully resolved, and a 504,492-particle stack containing all 2D classes with at least some well-resolved regions. An ab initio C2 symmetric 3D map was generated from the 78,577-particle stack, and automatic 3D refinement using C2 symmetry resulted in an initial intermediate resolution initial map. The 172,398-particle stack was exported to Relion, and the C2 symmetric initial map was used as a template for 3D auto-refinement, first in C2 and ultimately using C1 symmetry with C2 symmetry relaxation. This resulted in an intermediate resolution HCMV pentamer dimer map in which a single asymmetrically bound THBD was resolved at the dimer interface. Returning to cisTEM, the most inclusive 504,492 particle stack was then refined in C1 against this asymmetric map, using a full-molecule mask and also three different focused masks: one that included only THBD and UL128, UL130, UL131 of both protomers, one that included only gL, gH, 13H11, and MSL109 of a single protomer of the pseudodimer, and one that included only gL, gH, 13H11, and MSL109 of the other protomer. Phenix ResolveCryoEM density modification was applied to each of these, and the final map used for model building was generated by combining the density modified maps in UCSF Chimera by fitting the focused maps to the full-molecule map by correlation, resampling to a common grid, and taking the highest value between the three focused maps for each voxel (vop maximum). Local resolution was calculated from the unsharpened, unfiltered half maps using Relion postprocessing.

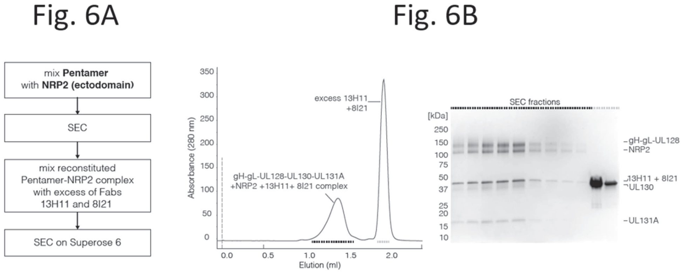

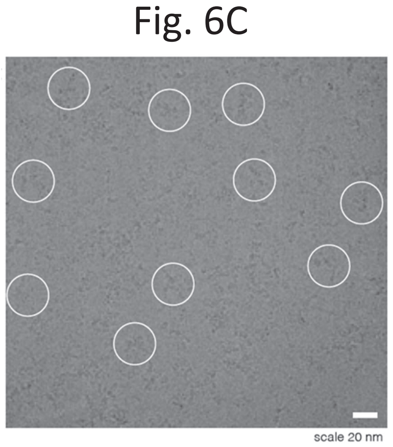

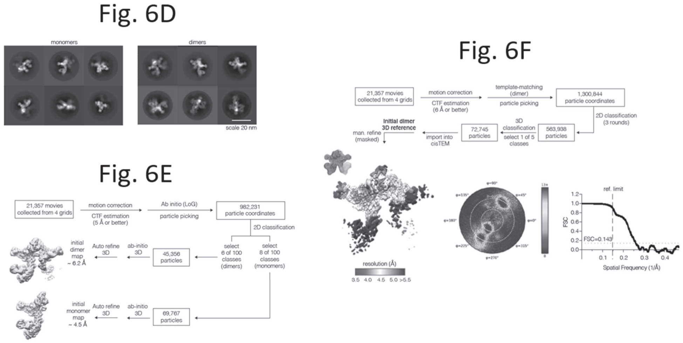

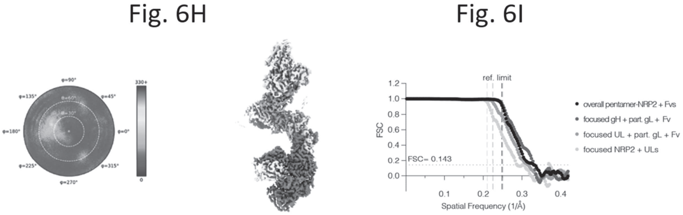

The HCMV pentamer-NRP2-13H11-8I21 cryoEM data were processed as described in Figs. 6A-6I. The 21,357 movies were motion corrected using Relion, and CTF parameters were estimated with CTFFIND4, and images with CTF fit resolutions of at least 5 Å were selected for further processing. Relion ab-inito Laplace-of-Gaussian particle picking resulted in 982,313 particle coordinates, which were extracted using a box size of 142 pixels and a pixel size of 3 Å. After 2D classification, 2D classes of apparent HCMV pentamers (69,767 particles) were re-extracted to a box size of 400 pixels and 1.07 Å per pixel, and subjected to Relion ab inito 3D refinement followed by 3D auto-refinement, resulting in an initial pentamer map. For the dimeric pentamer-NRP2-8I21-13H11, 2D class selections were used as templates for Relion template-based particle picking, resulting in 1,300,844 coordinates. These coordinates were extracted to a box size of 400 pixels and 1.07 Å per pixel, and subjected to hierarchical 2D classification and selection, ultimately resulting in 72,745 particles. These particles were imported directly to cisTEM, and refined against the initial dimeric pentamer map using a full molecule mask (no data beyond 6.7 Å were used in the refinement). For the monomeric pentamer-NRP2-8I21-13H11 complexes, 21,357 images with CTF fit resolutions of at least 6 Å were selected for further processing. A total of 2,532,206 particles were picked by template-matching with projections from 30 Å low-pass filtered single Pentamer-NRP2-8I21-13H11 reconstructions with gautomatch (MRC Laboratory of Molecular Biology). Particles were extracted using a box-size of 66 px and 5.4 Å pixel size, sorted using Relion 2D classification and 2,252,924 particles were extracted with a box size of 280 px and a px size of 1.35 Å. These particles were imported directly to cisTEM, and refined against the initial monomeric pentamer map using a full molecule mask (no data beyond 4 Å were used in the refinement). To improve the quality of the map, focused refinements were conducted after dividing the map into three distinct regions using masks and applying low-pass filter outside the mask (filter resolution 20 Å) and a score threshold of 0.20. Phenix ResolveCryoEM density modification was applied to each of these maps, and the final map used for model building was then generated by combining the density modified maps in UCSF Chimera. Local resolution was calculated from the unsharpened, unfiltered half maps using Relion postprocessing for the dimeric pentamer-NRP2-8I21-13H11. For the monomeric pentamer-NRP2-8I21-13H11 complex, local resolution was determined in cisTEM using a reimplementation of the blocres algorithm.

*v. Model building and structure analysis*

The gH and gL, UL128, UL130 and UL131A subunits of the HCMV pentamer structure (Chandramouli et al., Sci. Immunol., 2(30): 2017) were fit as a rigid body into the cryoEM map. The THBD Lectin domain was built based on a homology model of human RegIIalpha (PDB: 4MTH, (Mukherjee et al., Nature, 505(7481): 103-107, 2014)) using SwissModel (Waterhouse et al., Nucleic Acids Res., 46(W1): W296-W303, 2018). The structure of NRP2 (PDB: 2QQO) was used as a template for modeling of NRP2 a1-a2-b1b2 domains. The resulting model was fit as a rigid body into the cryoEM map. After extensive rebuilding and manual adjustments, multiple rounds of real space refinement using the phenix.real_space_refinement (Afonine et al., Acta Crystallogr. Sect. D Struct. Biol., 74(6): 531-544, 2018) tool was used to correct global structural differences between the initial model and the map. The model was further manually adjusted in Coot (Emsley et al., Acta Crystallogr. Sect. D Biol. Crystallogr., 66(4): 486-501, 2010) through iterative rounds of model building and real space refinements in Isolde (Croll, Acta Crystallogr. Sect. D Struct. Biol., D74: 519-530, 2018) and Phenix (Afonine et al., Acta Crystallogr. Sect. D Struct. Biol., 74(6): 531-544, 2018). The model was validated using phenix.validation_cryoem (Afonine et al., Acta Crystallogr. Sect. D Struct. Biol., 74(6): 531-544, 2018) with built-in MolProbity scoring (Williams et al., Protein Sci., 27(1): 293-315, 2018). Figures were made using PyMOL (The PyMOL Molecular Graphics System, v.2.07 Schrödinger, LLC) and UCSF ChimeraX (Goddard et al., Protein Sci., 27(1): 14-25, 2018). Sequences were aligned using Clustal Omega (Sievers et al., Mol. Syst. Biol., 7(1), 2011) within JalView (Waterhouse et al., Bioinformatics, 25(9): 1189-1191, 2009) and illustrated with ESPript 3.0 (Robert and Gouet, Nucleic Acids Res., 42(W1): W320-W324, 2014).

*vi. Cells and viruses*

Cells used for these studies were the same as described in Martinez-Martin et al., Cell, 174(5): 1158-1171, 2018. ARPE-19 is a male cell line derived from the spontaneously arising retinal pigment epithelia (RPE) cell line (ATCC CRL-2302) and was cultured in D-MEM/F-12 +GLUTAMAX™ (Thermo Fisher Scientific) supplemented with 10% FBS and penicillin/streptomycin (Thermo Fisher Scientific). HAP-1 cell is a male chronic myelogenous leukemia (CML) cell line derived from KBM-7 cell line, obtained from Horizon Genomics GmbH and cultured in Iscove's Modified Dulbecco's Medium (IMDM) + GLUTAMAX™ in the presence of 10% FBS and penicillin/streptomycin. Human Umbilical Vein Endothelial Cells (HUVEC, pool from male and female donors) were obtained from Lonza and provided by Fondazione IRCCS Policlinico San Matteo, Pavia, Italy, and were cultured in ENDOGRO™-VEGF Complete Media (Millipore). HEK293FT is a female human embryonic kidney cell line isolate derived from human embryonal kidney cells transformed with the SV40 large T antigen (Thermo Fisher Scientific). The HEK 293FT cell line was cultured in D-MEM+10% FBS, 0.1 mM MEM non-essential amino acids, GLUTAMAX™, 1 mM MEM sodium pyruvate and 500 mg/mL GENETICIN™ (Thermo Fisher Scientific). All cell lines were confirmed to be free of Mycoplasma. HCMV clinical isolate VR1814 was provided by Virologia e Microbiologia, Fondazione IRCCS Policlinico San Matteo, Pavia, Italy. HCMV VR1814 was propagated in HUVECs. ARPE-19 and HAP-1 cells were authenticated by analysis of Short tandem repeat (STR) loci at ATCC and Horizon Genomics GmbH, respectively. HUVEC were authenticated by expression of CD31/105, von Willebrand Factor VIII, and are positive for acetylated low-density lipoprotein uptake. HEK293FT were purchased from Thermo Fisher Scientific and authenticated by their ability to produce high titer infective lentiviral particles.

### vii. Flow cytometry staining

Flow cytometry staining was performed as described in Martinez-Martin et al., Cell, 174(5): 1158-1171, 2018. For cell-surface staining, adherent cells (ARPE-19, HUVEC and HAP-1) were gently detached with a cell scraper and washed twice with PBS+2% FBS, incubated in PBS + 0.5% BSA + 2 mM EDTA with specific anti-Neuropilin-2 and anti-CD46 antibodies (AF2215 and AF2005, R&D systems), anti-thrombomodulin antibody (clone 141C01, Abcam) or, as isotype control, normal sheep IgG affinity pure (R&D Systems) at 2 μg/ml for 30 minutes on ice. After two washes, cells were incubated with anti-mouse IgG (H+L) Alexa Fluor 594 conjugated (Thermo Fisher Scientific) secondary antibodies at 2 mg/ml for 30 minutes on ice, washed twice, and acquired with a FACS LSRFORTESSA™ (BD Biosciences) flow cytometer. Analysis was performed with FlowJo software (TreeStar).

### viii. Virus micro-neutralization

Serial dilutions of antibodies and soluble recombinant proteins were pre-incubated with an HCMV clinical isolate (strain VR1814) for 1 hour at 37°C and added to confluent monolayers of ARPE-19, HUVEC or HAP-1 cells cultured in 96-well flat-bottom plates (multiplicity of infection (MOI) of 1). Serial dilutions (1:3) were used for micro neutralization assays, with the first dilution at 20μg/mL. Infected cells were harvested after 3 days and fixed with BD CYTOFIX/CYTOPERM™ (Thermo Fisher Scientific), washed twice with PBS+2% FBS, and incubated in PBS + 0.5% BSA + 2 mM EDTA with the specific mouse anti-pp72 antibody (Clone 6E1, Santa Cruz Biotechnology, SC-69834) or isotype control at 2 μg/mL for 30 minutes on ice. After two washes, cells were incubated with goat anti-mouse IgG (H+L) Alexa Fluor 647 conjugated (Thermo Fisher Scientific) secondary antibodies at 2 μg/mL for 30 minutes on ice. Dead cells were excluded from counting by staining with 7-aminoactinomycin D (7-AAD; BioLegend). Samples were acquired with a FACS LSRFORTESSA™ (BD Biosciences) flow cytometer. Analysis was performed with FlowJo software (TreeStar). The percentage of infected cells was calculated by FACS. Dose-response curves were generated by plotting the relative infected cells against MFI signal. The concentration causing inhibition of 50% of infection (IC50) was calculated by nonlinear regression with Prism 8 (GraphPad Software).

### ix. Gene overexpression and gene KO

Gene overexpression and knockout (KO) for these studies were performed as described in Martinez-Martin et al., Cell, 174(5): 1158-1171, 2018. Human Nrp2 (Accession#AF022860), CD46 (Accession#NM_002389) and THBD (Accession#AF495471) were ordered from the GenEZ ORF database (Genscript) and cloned in pCDH-EF1-MCS (System Biosciences). Lentiviral particles were generated by PEI transfection of HEK293FT (Thermo Fisher Scientific) with pCDH-EF1-MCS (System Biosciences), pMD2.G (Addgene) and psPAX (Addgene) and purified on a sucrose cushion. HAP-1 KO by CRISPR/Cas9 of NRP2 was generated by Horizon Genomics GmbH. HAP-1 cells transduced with lentivirus were selected for 3 days with 0.5 mg/ml puromycin (Thermo Fisher Scientific). Single clones were expanded and effective KO of the target gene was analyzed by flow cytometry and confirmed by Sanger sequencing. The guide RNA used for CRISPR/Cas9 of NRP2 was

5'_GGGTAGTCCTGGGGGTAACC_3' (SEQ ID NO: 8).

### x. Spreading assay

To investigate cell spreading, HAP 1 cell monolayers were inoculated with HCMV VR1814 at an MOI of 0.1 PFU/cell (virus was titrated on ARPE-19 cells). Infected cell cultures were then stained at day 2 or 8 with mouse anti-pp72 antibody (Clone 6E1.Santa Cruz Biotechnology, SC-69834) or isotype control at 2μg/mL for 30 minutes on ice. Infected HAP1-Nrp2 KO were used as control and did not show the presence of any infection.

*xi. Biolayer interferometry*

The interactions between the NRP2, THBD, Fabs, and the HCMV pentamer were analyzed by biolayer interferometry using an OCTET® Red96 system (ForteBio) as previously described Kschonsak et al., Cell, 184(5): 1232-1244, 2021). All data were collected at 25°C using 96-well black flat bottom plates (Greiner Bio-One). Recombinant NRP2-Fc or THBD-Fc proteins were captured onto anti-human-Fc-coated (AHC) sensors (Sartorius), and tested for binding to the CMV pentamer as soluble analyte. Sensors were equilibrated for 10 minutes before the assay in PBS, following the manufacturer's instructions. All steps, including loading, baseline, association, and dissociation, were performed in PBS. For comparison of relative binding between the HCMV pentamer and the NRP2 WT and mutant proteins, the NRP2-Fc proteins were captured onto AHC sensors and tested for binding to the pentamer as soluble analyte at 50 nM concentration. Binding to NRP2 mutant proteins was represented relative to binding to the NRP2 WT protein. Binding units at the end of the association step were used to calculate the relative binding shown in the plot. For the NRP2-THBD competition experiment, THBD-Flag was added at different concentrations (1:1; 1:10; 1:50; 1:100 molar excess) relative to the pre-equilibrated pentamer-NRP2-Fc complex. For the Fab competition experiment, Fabs 2C12, 7I13, 8I21, 13H11 or MSL-109 were added in excess at a concentration of 100 μg/ml to the pre-equilibrated pentamer-NRP2-Fc complex. In all cases, data was acquired using the OCTET® Red96 instrument (Forte Pall software version 9.0). Subsequently, the Biaevaluation software version 4.1 (GE Healthcare) was utilized for calculations of kinetic parameters. Data were fit to a 1:1 Langmuir binding model.

*xii. Quantification and statistical analysis*

In Figs. 6A-6I, 9A-9D, 11A, and 11B, the resolution estimations of cryoEM density maps are based on the 0.143 Fourier Shell Correlation (FSC) criterion (Rosenthal and Henderson, J. Mol. Biol., 333(4): 721-745, 2003).
In Figs. 3A and 3B, statistical parameters including the exact value of n, precision measures (geometric mean $\pm$ SEM), and statistical significance are reported in the drawings and Brief Description of the Drawings. Data were judged to be statistically significant when $p < 0.05$. No statistical methods were used to predetermine sample size, no blinding of investigators was required, and no data points were excluded. Data were analyzed with Prism 8 (GraphPad Software) using the two-tailed non-parametric Mann-Whitney U test for two groups' comparison, or Kruskall-Wallis test (and Dunn's post-test) when three or more groups were compared (asterisks denote statistical significance *, $p < 0.05$; **, $p < 0.01$; ***, $p < 0.001$; ****, $p < 0.0001$). Cell surface Interaction Screen data were analyzed and represented using Microsoft Excel (version 14.7).

**Example 2. Structural basis for pentamer-NRP2 receptor binding**

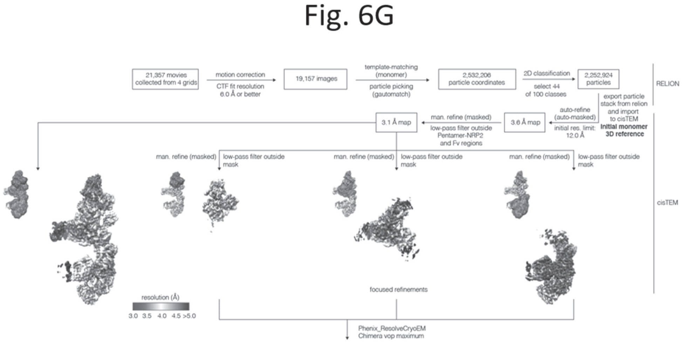

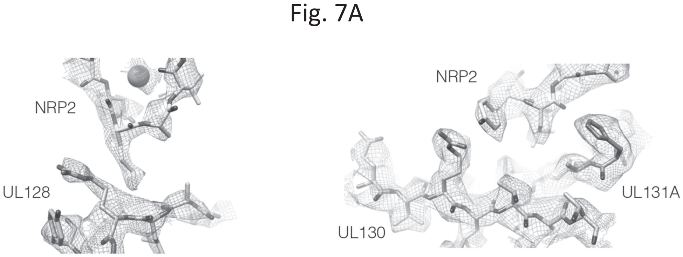

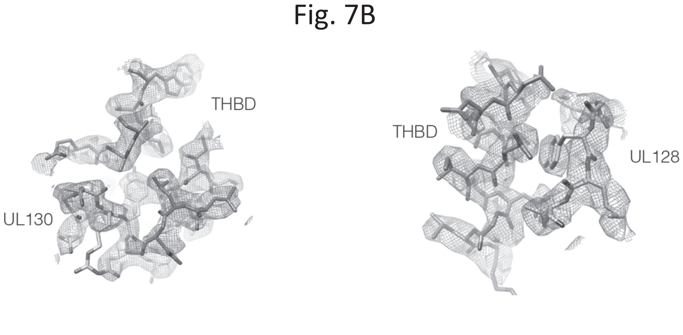

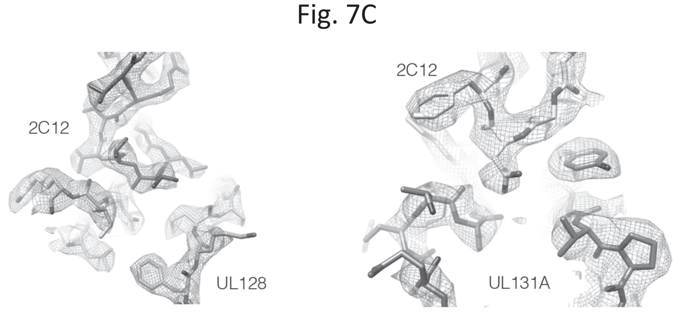

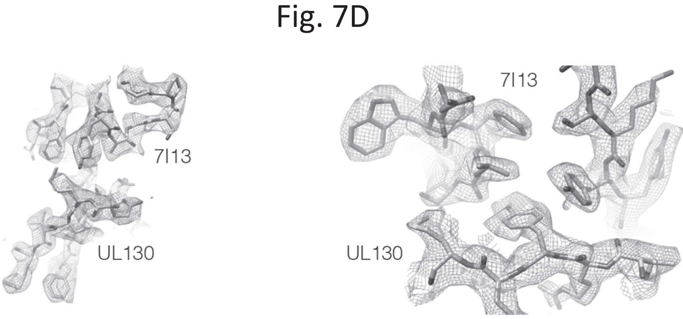

Prior structural studies of HCMV glycoprotein complexes have indicated that these molecules are refractory to high-resolution structure determination due to their inherent flexibility, elongated nature, and the numerous glycosylation sites (Ciferri et al., Proc. Natl. Acad. Sci., 112(6): 1767-1772, 2015). The HCMV pentamer was therefore reconstituted in complex with the soluble ectodomain of Neuropilin2 (NRP2) and the neutralizing Fabs 13H11 and 8I21, and single particle cryo-electron microscopy (cryo-EM) was used to determine their structure (Figs. 1A-1H and 6A-6I). Two-dimensional class averaging identified two particle populations. One population was monomeric, representing the majority of the particles in which one pentamer was bound to one copy of NRP2. Unexpectedly, a second dimeric population was identified in which two pentamer complexes were wrapped around a single NRP2 molecule and arranged in a head-to-head fashion (Figs. 6D-6F).
The structure of the monomeric pentamer-NRP2 complex bound to 13H11 and 8I21 was determined to a resolution of ~3.1 Å (Figs. 6G-6I). Focused 3D reconstructions allowed a structural model of the majority of the pentamer-13H11-8I21 complex and the NRP2 a2, b1 and b2 domains to be built. (Figs. 1C, 1D, and 7A and Table 1). Density for the a1 domain of NRP2 was weaker than the remaining part of the complex, presumably due to flexibility or to the ability of a1 domain to loosely interact with the pentamer in different orientations (Figs. 1D and 6G). No density was observed for the C-terminal MAM domain of NRP2, despite its presence in the protein construct and sample.

**Table 1. Cryo-EM data collection, refinement, and validation statistics**

| | HCMV Pentamer gHgLUL128 UL130UL131A + 2C12 +7113 + 13H11 | HCMV Pentamer gHgLUL128 UL130UL131A + NRP2 +8121 + 13H11 (monomer) | HCMV Pentamer gHgLUL128 UL130UL131A + NRP2 +8121 + 13H11 (dimer) | HCMV Pentamer gHgLUL128 UL130UL131A + THBD + MSL-109 + 13H11 (dimer) |
|---|---|---|---|---|
| **Data Collection** | | | | |
| Magnification | 165,000x | 165,000x | 165,000x | 165,000x |
| Voltage (kV) | 300 | 300 | 300 | 300 |
| Electron exposure (e/$Å^2$) | 58 | 52 | 52 | 64 |
| Defocus range ($\mu$m) | 0.5-1.5 | 0.5-1.5 | 0.5-1.5 | 0.5-1.5 |
| Pixel size (Å) | 0.824 | 0.824 | 0.824 | 0.832 |
| Symmetry imposed | C1 | C1 | C1 | C1 |
| Initial particle images | 5,128,264 | 2,532,206 | 1,300,8449 | 10,680,163 |
| Final particle images | 4,792,984 | 2,252,924 | 72,745 | 504,492 |
| Map resolution (Å) overall | 2.9 | 3.1 | 3.8 | 3.3 |
| FSC threshold | 0.143 | 0.143 | 0.143 | 0.143 |
| Map resolution range (Å) | 2.7-47.6 | 2.9-54.0 | 3.6-47.6 | 3.1-50.0 |
| **Refinement** | | | | |
| Initial models used (PDB code) | Pentamer: 5VOB | NRP2: 2QQO | | THBD homology model on 4MTH |
| Model resolution (Å) | 3.0 | 2.8 | | 3.4 |
| FSC threshold | 0.5 | 0.5 | | 0.5 |
| Model resolution range (Å) | 2.7-47.6 | 2.9-54.0 | | 3.1-50.0 |
| Map-sharpening B-factors ($Å^2$) | -90 | N/A | | N/A |
| **Model composition** | | | | |
| Non-hydrogen atoms | 15473 | 16413 | | 28,717 |
| Protein residues | 1946 | 2045 | | 3595 |
| Waters | 0 | 0 | | 0 |
| Ligands | 6 | 10 | | 14 |
| **B factors ($A^2$)** | | | | |
| Protein | 65.48 | 39.64 | | 64.77 |
| Ligand | 88.75 | 48.63 | | 71.87 |
| **R.m.s. deviations** | | | | |
| Bond lengths (Å) | 0.0004 | 0.005 | | 0.005 |

(continued)

| R.m.s. deviations | | | | |
|---|---|---|---|---|
| Bond angles (°) | 1.012 | 1.026 | | 1.027 |
| **Validation** | | | | |
| MolProbity score | 1.53 | 1.59 | | 1.72 |
| Clashscore | 4.99 | 7.35 | | 8.64 |
| Poor rotamers (%) | 0.00 | 0.00 | | 0.00 |
| **Ramachandran Plot** | | | | |
| Favored (%) | 96.08 | 96.87 | | 96.20 |
| Allowed (%) | 3.92 | 3.13 | | 3.80 |
| Disallowed (%) | 0.00 | 0.00 | | 0.00 |

Three main sites of interaction between NRP2 and the pentamer were identified, in agreement with previous observations (Wrapp et al., bioRxiv, p. 2021.03.25.436804, 2021) (Figs. 1E-1G). At Site 1, a calcium coordinating region in the a2 domain of NRP2 establishes charge interactions with residues localized in the N-terminal domain of UL128 of the pentamer (Figs. 1E-1G). Specifically, NRP2-D197 and NRP2-D252 interact with UL128-K47 while NRP2-N172 contacts UL128-R57 (Fig. 1E). Site 2 is established between the b2 domain of NRP2 and the C-terminal strand of UL130 and UL131A (Figs. 1F and 1G). Notably, it was found that charge reversal mutations on the pentamer at either Site 1 or Site 2 disrupted NRP2 binding (Fig. 1H). Relative to unbound NRP2 structures, the a1 domain becomes displaced from the a2b1b2 core upon pentamer binding and is repositioned to a third site of interaction, Site3, housed along a concave surface of the UL components at the site of attachment between UL128 and gL (Fig. 1D). This region was poorly resolved in the cryoEM map, suggesting that a1 domain binding to monomeric pentamer is dynamic, consistent with the finding that NRP2 is still able to interact with the pentamer in the absence of the a1 domain, albeit to a lower degree (Wrapp et al., bioRxiv, p. 2021.03.25.436804, 2021).

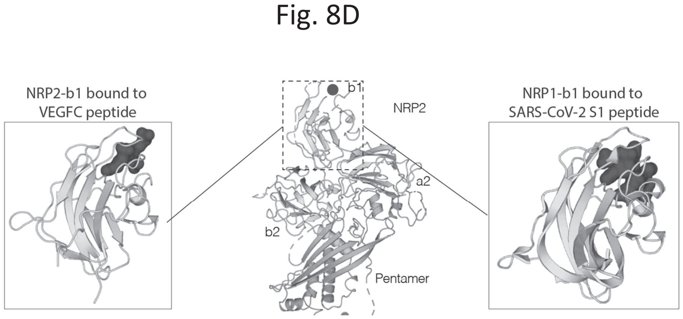

Although the folds of NRP1 and NRP2 are conserved, the sites of NRP2 interaction with HCMV Pentamer are divergent in NRP1, which provides a structural rationale for the specificity of the NRP2 receptor (Figs. 8A-8C; Appleton et al., EMBO J., 26: 4902-4912, 2007). Interestingly, NRP1 and NRP2 proteins are known to interact with several common partners, including VEGF and the spike protein of SARS-CoV-2, through a mode in which their respective b1 domains interact with a [R/K]XX[R/K] (CendR) motif in the binding partner (Daly et al., Science, 370(6518): 861-865, 2020; Parker et al., J. Biol. Chem., 287(14): 11082-11089, 2012). However, the structure presented herein and studies from the McLellan group (Wrapp et al., bioRxiv, p. 2021.03.25.436804, 2021) unambiguously demonstrate that the interaction between the pentamer and NRP2 does not involve this common binding surface on the b1 domain, but rather requires specific interactions between the a2 and b2 domains of NRP2 and the UL components of the pentamer, and in particular UL128 and UL131A (Figs. 1E-1G and 8D; Daly et al., Science, 370: 861-865, 2020; Parker et al., J. Biol. Chem., 287: 11082-11089, 2012). These features highlight the structural economy underlying the diverse array of cellular proteins with which the pentamer can interact.

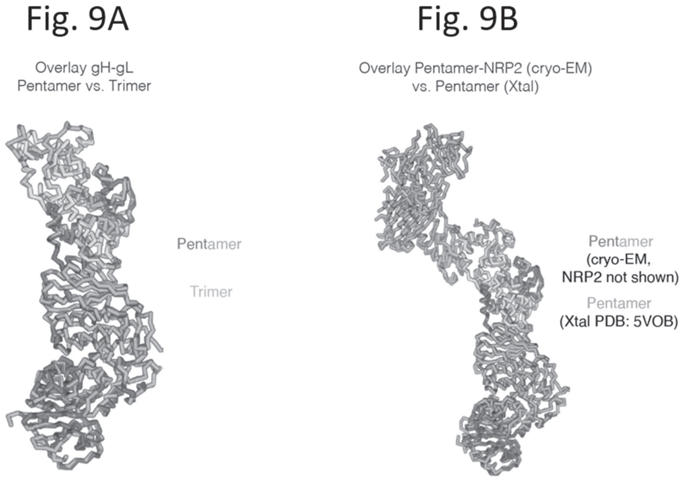

The NRP2 receptor interaction with the pentamer may propagate a conformational change to enable or disable binding of other HCMV glycoproteins, such as the prefusion gB. The cryo-EM structure of the pentamer bound to NRP2 was compared with the previously determined crystal structure of the pentamer in absence of a receptor (PDB:5VOB (Chandramouli et al., Sci. Immunol., 2(30): 2017)) as well as the structure of the HCMV gHgLgO trimer (gH and gL subunits only (Kschonsak et al., Cell, 184(5): 1232-1244, 2021). Notably, and in agreement with previous findings (Wrapp et al., bioRxiv, p. 2021.03.25.436804, 2021), very similar conformations of gH/gL are observed across all comparisons (Figs. 9A and 9B), implying that a different mechanism must underlie the activation of the HCMV fusion machinery, as discussed below.

## Example 3. Structural basis for pentamer-THBD receptor binding

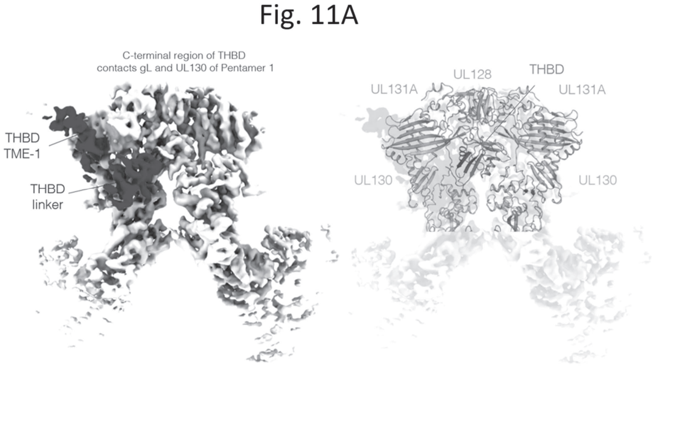

Next, a stoichiometric complex of the soluble THBD ectodomain with the HCMV pentamer and the Fabs 13H11 and MSL-109 was reconstituted and the structure was determined to an overall resolution of ~3.3 Å (Figs. 2A-2D, 7B, and 10A-10F and Table 1). Reminiscent of the NRP2 head-to-head pentamer dimer population, two-dimensional classification revealed two pentamer molecules wrapped around one THBD receptor in a head-to-head fashion interacting through the UL128 subunit (Fig. 2B). In this dimeric complex structure, the THBD N-terminal lectin-like domain is sandwiched in between two pentamer molecules, and specifically, wedged in between the two concave surfaces formed by the UL components at the site of interaction between UL128 and gL (Fig. 2B). Additional low-resolution density was observed in the direction of the anchoring point of the receptor on the host cell membrane, likely corresponding to the THBD TME1 and the linker region connecting it to the THBD lectin-like domain on the convex surface of the pentamer formed by the UL130 and UL131A subunits (Fig. 11A). Binding of THBD did not induce any major structural rearrangement of the pentamer (Supplementary Figure S4D), as observed for NRP2-pentamer complex (Fig. 9C).

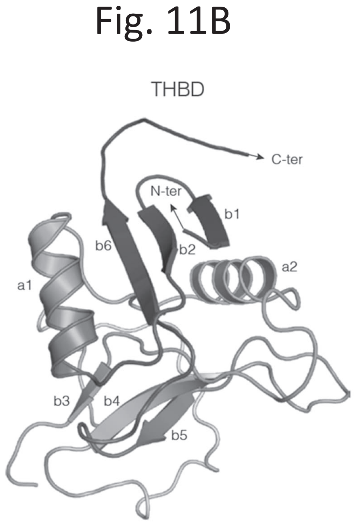

The THBD lectin-like domain is a globular domain comprised of six β strands (b1-b6) and two α helices (a1 and a2) inserted between b2 and b3 (Fig. 11B). Two opposite surfaces of THBD are recognized by the same regions of the two engaging pentamer molecules, uncovering a remarkable structural plasticity of this interface (Fig. 2B). Specifically, the N-terminal region of UL128 (UL128-R42 or UL128-Y44) interacts with either THBD-R83 and THBD-E154 or THBD-A123 and THBD-L125, respectively (Fig. 2B). Similarly, the hairpin of UL130 centered at residues UL130-Y169 interacts on one side with THBD-S149 and on the other side with THBD-C133 (Fig. 2B). Additionally, the C-terminal region of pentamer 1 UL128 (UL128-R131, UL128-N134, UL128-Y137) interacts with THBD-V66 and THBD-D69 while a distinct region on pentamer 2 UL128 (UL128-R158, UL128-R163, UL128-Y168) contacts THBD-S49 and THBD-D53 (Fig. 2B). Overall, this structure reveals a surprisingly large and polyspecific interaction footprint between THBD and a pentamer dimer at overlapping regions on the UL128 and UL130 subunits (Figs. 2C and 2D).

**Example 4. THBD is a functional receptor for HCMV and competes with NRP2 for pentamer binding**

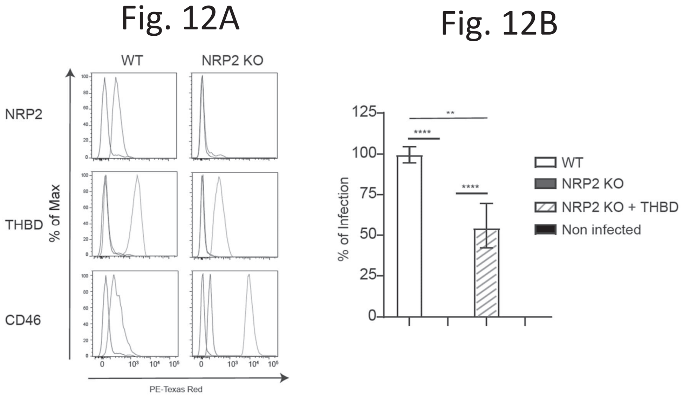

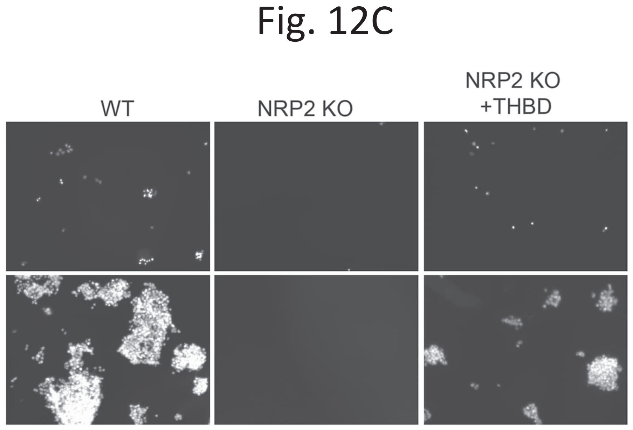

To determine whether THBD is a functional receptor for HCMV, the ability of soluble proteins and antibodies to inhibit viral entry in epithelial and endothelial cells was tested. As expected, recombinant NRP2 and a pan-NRP antibody (Appleton et al., EMBO J., 26(23): 4902-4912, 2007) inhibited HCMV infection in epithelial and endothelial cells in a dose-dependent manner (Fig. 3A; Martinez-Martin et al., Cell, 174(5): 1158-1171, 2018). Recombinant THBD reduced viral infection to a much lower extent, but a combination of recombinant THBD and NRP2 inhibited HCMV entry to higher levels than NRP2 alone, suggesting a potential functional role for THBD in HCMV infection (Fig. 3A), and in agreement with published results (Martinez-Martin et al., Cell, 174(5): 1158-1171, 2018). To further test this hypothesis, the ability of THBD to mediate HCMV entry in cells lacking NRP2 was examined (Figs. 3B and 12A). THBD overexpression had minimal effect on HCMV infection of HAP-1 wild-type (WT), but its overexpression in the NRP2 knockout (KO) cells dramatically increased viral entry (Figs. 3B, 12A, and 12B). As a control, the experiment was repeated in the presence of 8121 mAb at 0.1μg/mL. Addition of the pentamer-specific mAb completely abolished infection, confirming a pentamer-specific infection (Fig. 3B). Moreover, it was observed that THBD-expressing cells were able to spread the virus (Fig. 12C). Taken together, these data indicate that both NRP2 and THBD are relevant receptors for HCMV that may function as independent receptors or co-receptors.

These structural analyses suggest how the pentamer has evolved to engage architecturally distinct receptors localized on the surface of different cells. Notably, NRP2 and THBD bind to the pentamer through unique interaction surfaces, where superposition of the pentamer-NRP2 and pentamer-THBD complexes demonstrates that these receptors share an overlapping binding site on the pentamer (Fig. 3C). Specifically, the common interaction site is localized at the interface where UL128 engages gL and is responsible for the binding of NRP2 a1 domain and the THBD N-terminal Lectin domain, indicating that these interacting partners cannot bind the pentamer at the same time (Fig. 3C). To test the observation, competition experiments were performed by incubating HCMV pentamer bound to NRP2 with increasing amounts of THBD, and it was observed that THBD can compete for NRP2 binding at high concentrations (Fig. 3D). Overall, these structural and biophysical data indicate that NRP2 and THBD do not function as co-receptors, but rather mediate HCMV tropism by acting as independent receptors.

**Example 5. HCMV pentamer dimers engage NRP2 and THBD in a similar architecture**

The unexpected dimeric architecture of the pentamer bound to NRP2 is highly similar to the dimeric population observed upon THBD binding (Fig. 3E). The a1 domain of NRP2 is encased between two pentamer molecules, analogous to the N-terminal lectin domain of THBD, whereas the a2b1b2 domains of NRP2 engages only one of the pentamers to form a markedly asymmetric assembly (Fig. 3E). Notably, these two receptor super-assemblies also share a conserved dimerization interface mediated by a network of residues at the N-terminal region of the UL128 subunit localized at the most distal region of the pentamer (Figs. 3F and 3G), suggesting a potential physiological relevance of the dimeric assembly.

**Example 6. Structural basis for pentamer neutralization by monoclonal antibodies**

Highly potent neutralizing antibodies against HCMV have been isolated from immortalized memory B-cells of HCMV-immune donors and have been shown to target conformational epitopes of the pentamer (Macagno et al., J. Virol.,

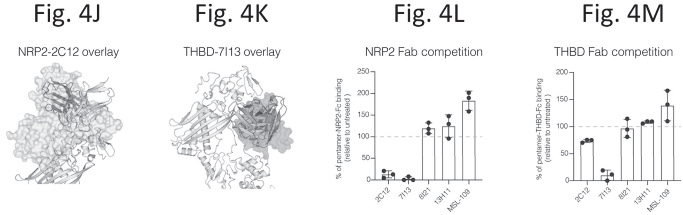

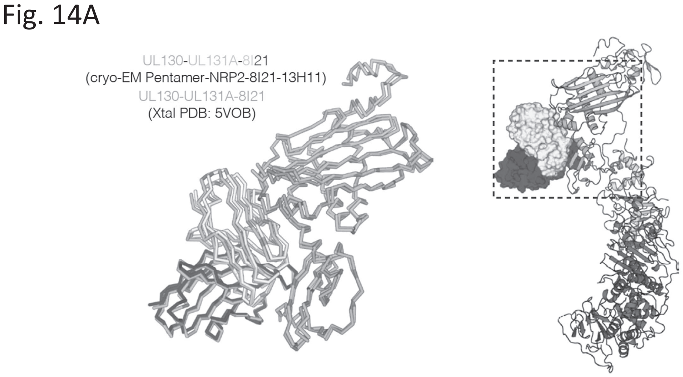

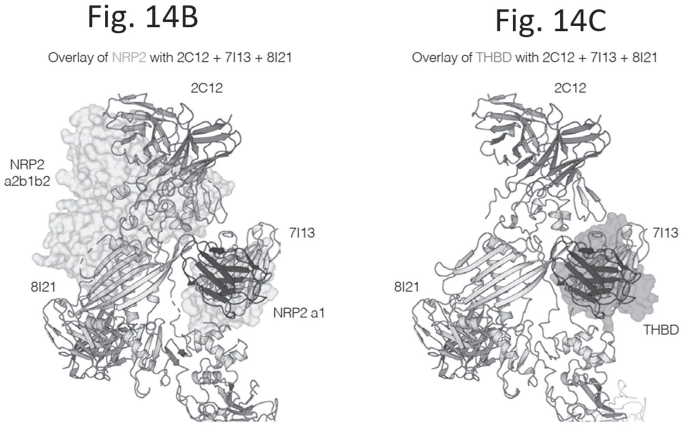

84(2): 1005-1013, 2010; Ciferri et al., PLoS Pathog., 11(10), 2015). To provide a view of these neutralizing interactions, the structure of the pentamer bound to the highly potent neutralizing Fabs 2C12, and 7I13, as well as bound to the gH binding Fab 13H11, was determined to an overall resolution of ~2.9 Å (Figs. 13A-13H). Remarkably, 2C12 binds the UL131A and UL128 subunits of the pentamer in the same area recognized by NRP2 a2b1b2 domains (Figs. 4A-4E and 4J). Specifically, Y55, Y116, G119 and N120 from the light chain of 2C12 bind the N-terminal region of UL131A between the residues T24-N28, while the heavy chain of 2C12 (N81, Q125 and V130) recognizes UL131A-K27 and UL128-R51, R108 and 1109 (Figs. 4B-4E). Fab 7113 buries a large footprint (~1,200Å$^2$) and binds to the same surface on the pentamer that recognizes THBD and the NRP2 a1 domain (Figs. 4E-4I and 4K). Residues from both light and heavy chains of 7113 contact UL128 on residues Y44, P59, R131, N134 and L135, and bind UL130 at R168, Y169, M171 and N200, through a combination of hydrophobic, polar and charge interactions (Figs 4F-4I). Fab 8I21, shown in the structure of the pentamer-NRP2 complex (Figs. 6A-6I), recognizes the pentamer in a region non-overlapping with THBD or NRP2 binding sites (Figs. 4A and 14A-14C). Superposition of the 2C12-7I13-pentamer and NRP2-pentamer or THBD-pentamer structures indicate that 2C12 blocks NRP2 binding, but not THBD binding (Figs. 4J and 14B), and a comparison of these structures suggests 7I13 can block binding of both receptors, NRP2 and THBD (Figs. 4K and 14C). In fact, competition interaction experiments to the pentamer show that NRP2 binding is blocked by 2C12 and 7113, but not by 8I21 or gH-specific neutralizing antibodies (Fig. 4L), whereas THBD binding is blocked by 7113 but not by 2C12, 8I21 or gH specific neutralizing antibodies (Fig. 4M).

### Example 7. Identification of beta-2-microglobulin as a novel HCMV pentamer interactor

Beta-2 microglobulin (B2M) was also discovered as an additional interaction partner, and its structure in complex with the HCMV pentamer was determined.

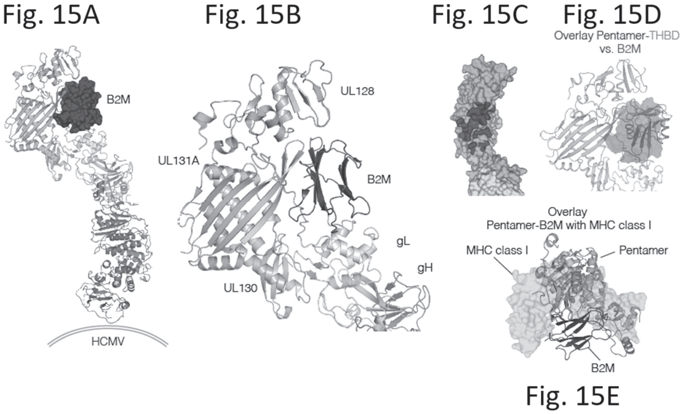

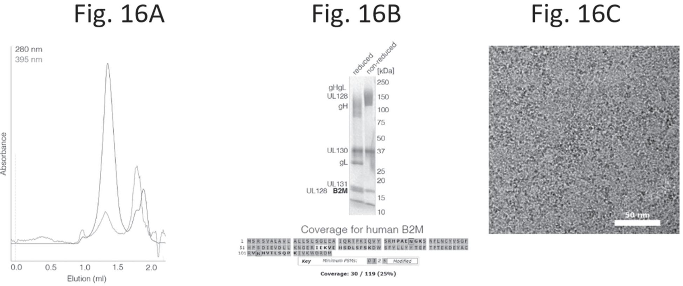

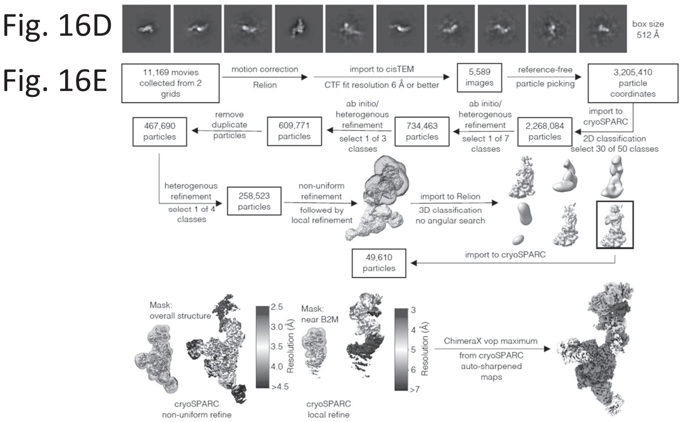

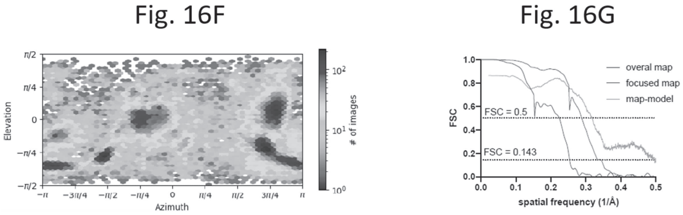

A prior genome-wide CRISPR/Cas9 screen identified olfactory receptor (OR14L1) as a putative receptor for the HCMV pentamer, and a synthetic peptide comprising the N-terminal 26 residues of OR14L1 was suggested as the minimal region required for interaction (E et al., Proc. Natl. Acad. Sci. U.S.A., 116: 7043-7052, 2019). Since ORL4L1 is a multi-transmembrane G-protein coupled receptor (GPCR) belonging to the olfactory receptor subfamily, reconstitution of the HCMV pentamer with the reported OR14L1 minimal peptide fused to GFP was attempted and non-stoichiometric binding was observed (Figs. 16A and 16B). Cryo-EM and multiple rounds of 3D-classification were used in the attempt to isolate particles bound to the OR14L1 peptide to determine its structure (Figs. 16C-16G). Although no additional density accounting for the OR14L1 peptide-GFP fusion was observed (data not shown), a subset of particles (~10%) showing additional density (at ~ 3.5Å resolution) that corresponded to a beta-sandwich fold at the concave interface of the UL subunits was identified (Fig. 16E). SDS-PAGE and mass spectrometry analysis indicated that the sample contained an abundant additional co-purifying protein, beta-2-microglobulin (B2M), a 11.8 kDa cellular protein present in body fluids and tissue culture media (Fig. 16B). Although its presence in the cryoEM map was unexpected, B2M was previously shown to bind to HCMV viral particles and increase HCMV ability to infect cells (Grundy et al., J. Gen. Virol., 68(3): 793-803, 1987). Rigid body refinement was used to confidently place the beta-sandwich fold determined from the prior crystal structures of B2M into this density (Figs. 15A and 15B, Smith et al., Immunity, 4: 215-228, 1996). This structure reveals that B2M contacts the same regions on UL128 and UL130 recognized by THBD, further demonstrating the structural plasticity of this region on the pentamer (Figs. 15C and 15D).

B2M normally binds non-covalently with the $\alpha$3 domain of the major histocompatibility complex (MHC) class I to contribute to cell-mediated immune responses against viral infections and malignant transformation (Li et al., Chin. Med. J. (Engl.), 129: 448-455, 2016), but the present structural analysis indicates that binding between B2M and the pentamer is incompatible with simultaneous binding to MHC class I (Fig. 15E).

Although the foregoing invention has been described in some detail by way of illustration and example for purposes of clarity of understanding, the descriptions and examples should not be construed as limiting the scope of the invention. The disclosures of all patent and scientific literature cited herein are expressly incorporated in their entirety by reference.

Aspect 1 is a method of identifying a modulator of the interaction between the human cytomegalovirus (HCMV) gH/gL/UL128-131A pentamer and beta-2-microglobulin (B2M), the method comprising:

(a) providing a candidate modulator;
(b) contacting the HCMV gH/gL/UL128-131A pentamer with B2M in the presence or absence of the candidate modulator under conditions permitting the binding of the HCMV gH/gL/UL128-131A pentamer to B2M; and
(c) measuring the binding of the HCMV gH/gL/UL 128-131A pentamer to B2M, wherein an increase or decrease in binding in the presence of the candidate modulator relative to binding in the absence of the candidate modulator identifies the candidate modulator as a modulator of the interaction between the HCMV gH/gL/UL128-131A pentamer and B2M.

Aspect 2 is a method of identifying a modulator of a downstream activity of the HCMV gH/gL/UL128-131A pentamer, the method comprising:

(a) providing a candidate modulator;
(b) contacting the HCMV gH/gL/UL128-131A pentamer with B2M in the presence or absence of the candidate modulator under conditions permitting the binding of the HCMV gH/gL/UL128-131A pentamer to B2M; and
(c) measuring a downstream activity of the HCMV gH/gL/UL128-131A pentamer, wherein a change in the downstream activity in the presence of the candidate modulator relative to the downstream activity in the absence of the candidate modulator identifies the candidate modulator as a modulator of the downstream activity of the HCMV gH/gL/UL128-131A pentamer.

Aspect 3 is a method of identifying a modulator of a downstream activity of B2M, the method comprising:

(a) providing a candidate modulator;
(b) contacting B2M with the HCMV gH/gL/UL128-131A pentamer in the presence or absence of the candidate modulator under conditions permitting the binding of B2M to the HCMV gH/gL/UL128-131A pentamer; and
(c) measuring a downstream activity of B2M, wherein a change in the downstream activity in the presence of the candidate modulator relative to the downstream activity in the absence of the candidate modulator identifies the candidate modulator as a modulator of the downstream activity of B2M.

Aspect 4 is the method of aspect 1, wherein the increase or decrease in binding is at least 50%, as measured by surface plasmon resonance, biolayer interferometry, or an enzyme-linked immunosorbent assay (ELISA).

Aspect 5 is the method of any one of aspects 1-4, wherein the modulator is an inhibitor of the downstream activity of the HCMV gH/gL/UL 128-131A pentamer or B2M.

Aspect 6 is the method of aspect 2 or 3, wherein the change in the downstream activity is a decrease in the amount, strength, or duration of the downstream activity.

Aspect 7 is the method of any one of aspects 1-6, wherein the modulator is a small molecule, an antibody or antigen-binding fragment thereof, a peptide, a mimic, or an inhibitory nucleic acid.

Aspect 8 is the method of aspect 7, wherein the inhibitory nucleic acid is an ASO or an siRNA.

Aspect 9 is the method of aspect 7, wherein the antigen-binding fragment is a bis-Fab, an Fv, a Fab, a Fab'-SH, a F(ab')2, a diabody, a linear antibody, an scFv, an scFab, a VH domain, or a VHH domain.

Aspect 10 is the method of aspect 7 or 9, wherein the antibody or antigen-binding fragment thereof binds the HCMV gH/gL/UL128-131A pentamer.

Aspect 11 is the method of aspect 7 or 9, wherein the antibody or antigen-binding fragment thereof binds B2M.

Aspect 12 is the method of any one of aspects 2-11, wherein the downstream activity is infection of a cell by HCMV.

Aspect 13 is the method of aspect 12, wherein infection is decreased in the presence of the modulator.

Aspect 14 is the method of aspect 13, wherein infection is decreased by at least 40%, as measured in a viral infection assay or a viral entry assay using pseudotyped particles.

Aspect 15 is the method of any one of aspects 1-14, wherein the modulator is an antibody or antigen-binding fragment thereof that binds the HCMV gH/gL/UL128-131A pentamer.

Aspect 16 is the method of any one of aspects 1-15, wherein the modulator is an antibody or antigen-binding fragment thereof that binds B2M.

Aspect 17 is a modulator of the interaction between the human cytomegalovirus (HCMV) gH/gL/UL 128-131A pentamer and neuropilin 2 (NRP2) that causes a decrease in the binding of the gH/gL/UL128-131A pentamer to NRP2, wherein the modulator binds to:

(a) one or more of residues D197, D252, N172, M253, Y458, and L459 of NRP2;
(b) one or both of residues K47 and R57 of the UL128 subunit of the gH/gL/UL128-131A pentamer;
(c) residue R193 of the UL130 subunit of the gH/gL/UL128-131A pentamer; and/or
(d) one or both of residues A114 and A117 of the UL131A subunit of the gH/gL/UL128-131A pentamer.

Aspect 18 is the modulator of aspect 17, wherein the modulator binds to:

(a) all six of residues D197, D252, N172, M253, Y458, and L459 of NRP2;
(b) both of residues K47 and R57 of the UL128 subunit of the gH/gL/UL128-131A pentamer;
(c) residue R193 of the UL130 subunit of the gH/gL/UL128-131A pentamer; and/or
(d) both of residues A114 and A117 of the UL131A subunit of the gH/gL/UL128-131A pentamer.

Aspect 19 is the modulator of aspect 17 or 18, wherein the modulator decreases binding of the gH/gL/UL128-131A pentamer to NRP2 by at least 50%.

Aspect 20 is the modulator of aspect 19, wherein the modulator decreases binding of the gH/gL/UL128-131A pentamer to NRP2 by at least 90%.

Aspect 21 is a modulator of the interaction between the HCMV gH/gL/UL128-131A pentamer and thrombomodulin (THBD) that causes a decrease in the binding of the gH/gL/UL128-131A pentamer to THBD, wherein the modulator binds to:

(a) one or more of residues S49, D53, V66, D69, R83, C96, E154, A123, L125, S149, and C133 of THBD;
(b) one or more of residues R42, Y44, R131, N134, Y137, R158, R163, and Y168 of the UL128 subunit of the gH/gL/UL128-131A pentamer; and/or
(c) one or more of residues N164, Y169, and M171 of the UL130 subunit of the gH/gL/UL128-131A pentamer.

Aspect 22 is the modulator of aspect 21, wherein the modulator binds to:

(a) all eleven of residues S49, D53, V66, D69, R83, C96, E154, A123, L125, S149, and C133 of THBD;
(b) all eight of residues R42, Y44, R131, N134, Y137, R158, R163, and Y168 of the UL128 subunit of the gH/gL/UL128-131A pentamer; and/or
(c) all three of residues N164, Y169, and M171 of the UL130 subunit of the gH/gL/UL128-131A pentamer.

Aspect 23 is the modulator of aspect 21 or 22, wherein the modulator decreases binding of the gH/gL/UL128-131A pentamer to THBD by at least 50%.

Aspect 24 is the modulator of aspect 23, wherein the modulator decreases binding of the gH/gL/UL128-131A pentamer to THBD by at least 90%.

Aspect 25 is a modulator of the interaction between the human cytomegalovirus (HCMV) gH/gL/UL 128-131A pentamer and beta-2-microglobulin (B2M) that causes a decrease in the binding of the gH/gL/UL128-131A pentamer to B2M.

Aspect 26 is the modulator of aspect 25, wherein the modulator decreases binding of the gH/gL/UL128-131A pentamer to B2M by at least 50%.

Aspect 27 is the modulator of aspect 26, wherein the modulator decreases binding of the gH/gL/UL128-131A pentamer to THBD by at least 90%.

Aspect 28 is the modulator of any one of aspects 19, 20, 23, 24, 26, and 27, wherein the decrease in binding is measured by surface plasmon resonance, biolayer interferometry, or an enzyme-linked immunosorbent assay (ELISA).

Aspect 29 is the modulator of any one of aspects 17-28, wherein the modulator causes a decrease in infection of a cell by HCMV relative to infection in the absence of the modulator.

Aspect 30 is the modulator of aspect 29, wherein infection is decreased by at least 40%, as measured in a viral infection assay or a viral entry assay using pseudotyped particles.

Aspect 31 is the modulator of any one of aspects 17-30, wherein the modulator is a small molecule, an antibody or antigen-binding fragment thereof, a peptide, a mimic, or an inhibitory nucleic acid.

Aspect 32 is the modulator of aspect 31, wherein the inhibitory nucleic acid is an antisense oligonucleotide (ASO) or an siRNA.

Aspect 33 is the modulator of aspect 31, wherein the antigen-binding fragment is a bis-Fab, an Fv, a Fab, a Fab'-SH, a $F(ab')_2$, a diabody, a linear antibody, an scFv, an scFab, a VH domain, or a VHH domain.

Aspect 34 is the modulator of aspect 31, wherein the antibody is a bispecific antibody or a multispecific antibody.

Aspect 35 is the modulator of any one of aspects 17-34, further comprising a pharmaceutically acceptable carrier.

Aspect 36 is a method for treating an HCMV infection in an individual, the method comprising administering to the individual an effective amount of the modulator of any one of aspects 17-35, thereby treating the individual.

Aspect 37 is the method of aspect 36, wherein the duration or severity of HCMV infection is decreased by at least 40% relative to an individual who has not been administered the modulator.

Aspect 38 is a method for preventing an HCMV infection in an individual, the method comprising administering to the individual an effective amount of the modulator of any one of aspects 17-35, thereby preventing an HCMV infection in the individual.

Aspect 39 is the method of prophylaxis against a secondary HCMV infection in an individual, the method comprising administering to the individual an effective amount of the modulator of any one of aspects 17-35, thereby preventing a secondary HCMV infection in the individual.

Aspect 40 is the method of aspect 39, wherein the secondary infection is an HCMV infection of an uninfected tissue.

Aspect 41 is the method of any one of aspects 36-40, wherein the individual is immunocompromised, is pregnant, or is an infant.

Aspect 42 is the use of the modulator of any one of aspects 17-35 in the manufacture of a medicament for treating an HCMV infection in an individual.

Aspect 43 is the use of the modulator of any one of aspects 17-35 in the manufacture of a medicament for preventing an HCMV infection in an individual.

Aspect 44 is the use of the modulator of any one of aspects 17-35 in the manufacture of a medicament for prophylaxis against a secondary HCMV infection in an individual.

Aspect 45 is the use of aspect 44, wherein the secondary infection is an HCMV infection of an uninfected tissue.

Aspect 46 is the use of any one of aspects 42-45, wherein the individual is immunocompromised, is pregnant, or is an infant.

Aspect 47 is the modulator of any one of aspects 17-35 for use in a method of treating an HCMV infection in an individual, wherein the method comprises administering to the individual an effective amount of the modulator of any one of aspects 17-35, thereby treating the individual.

Aspect 48 is the modulator of any one of aspects 17-35 for use in a method of preventing an HCMV infection in an individual, wherein the method comprises administering to the individual an effective amount of the modulator of any one of aspects 17-35, thereby preventing an HCMV infection in the individual.

Aspect 49 is the modulator of any one of aspects 17-35 for use in a method of prophylaxis against a secondary HCMV infection in an individual, wherein the method comprises administering to the individual an effective amount of the modulator of any one of aspects 17-35, thereby preventing a secondary HCMV infection in the individual.

Aspect 50 is the modulator for use of aspect 49, wherein the secondary infection is an HCMV infection of an uninfected tissue.

Aspect 51 is the modulator for use of any one of aspects 47-50, wherein the individual is immunocompromised, is pregnant, or is an infant.

**Claims**

1. A modulator of the interaction between the human cytomegalovirus (HCMV) gH/gL/UL128-131A pentamer and beta-2-microglobulin (B2M) that causes a decrease in the binding of the gH/gL/UL128-131A pentamer to B2M, in particular wherein the modulator decreases binding of the gH/gL/UL128-131A pentamer to B2M by at least 50%, more particularly wherein the modulator decreases binding of the gH/gL/UL128-131A pentamer to THBD by at least 90%.

2. The modulator of claim 1, wherein the decrease in binding is measured by surface plasmon resonance, biolayer interferometry, or an enzyme-linked immunosorbent assay (ELISA).

3. The modulator of claim 1, wherein the modulator causes a decrease in infection of a cell by HCMV relative to infection in the absence of the modulator, in particular , wherein infection is decreased by at least 40%, as measured in a viral infection assay or a viral entry assay using pseudotyped particles.

4. The modulator of claim 1, wherein the modulator is a small molecule, an antibody or antigen-binding fragment thereof, in particular wherein the antigen-binding fragment is a bis-Fab, an Fv, a Fab, a Fab'-SH, a F(ab')$_2$, a diabody, a linear antibody, an scFv, an scFab, a VH domain, or a VHH domain and/or in particular wherein the antibody is a bispecific antibody or a multispecific antibody, a peptide, a mimic, or an inhibitory nucleic acid, in particular wherein the inhibitory nucleic acid is an antisense oligonucleotide (ASO) or an siRNA.

5. The modulator of any one of claims 1-4, further comprising a pharmaceutically acceptable carrier.

6. A method for treating an HCMV infection in an individual, the method comprising administering to the individual an effective amount of the modulator of any one of claims 1-4, thereby treating the individual, in particular wherein the duration or severity of HCMV infection is decreased by at least 40% relative to an individual who has not been administered the modulator and/or in particular wherein the individual is immunocompromised, is pregnant, or is an infant.

7. A method for preventing an HCMV infection in an individual, the method comprising administering to the individual an effective amount of the modulator of any one of claims 1-4, thereby preventing an HCMV infection in the individual, in particular wherein the individual is immunocompromised, is pregnant, or is an infant.

8. A method of prophylaxis against a secondary HCMV infection in an individual, the method comprising administering to the individual an effective amount of the modulator of any one of claims 1-4, thereby preventing a secondary HCMV infection in the individual, in particular wherein the secondary infection is an HCMV infection of an uninfected tissue and/or in particular wherein the individual is immunocompromised, is pregnant, or is an infant.

9. Use of the modulator of any one of claims 1-5 in the manufacture of a medicament for treating or preventing an HCMV infection in an individual, or for prophylaxis against a secondary HCMV infection in an individual, in particular wherein the secondary infection is an HCMV infection of an uninfected tissue; in particular wherein the individual is immunocompromised, is pregnant, or is an infant.

10. The modulator of any one of claims 1-5 for use in a method of treating or preventing an HCMV infection in an individual or for use in a method of prophylaxis against a secondary HCMV infection in an individual, in particular wherein the secondary infection is an HCMV infection of an uninfected tissue, wherein the method comprises administering to the individual an effective amount of the modulator of any one of claims 1-5, thereby treating the individual; in particular wherein the individual is immunocompromised, is pregnant, or is an infant.

## Fig. 1A

○ Merlin  ◇ VR1814

Norm. binding signal of HCMV pentamer (% of max)

NRP1    NRP2

## Fig. 1C

NRP2

HCMV

## Fig. 1B

28    142 149    267 277    427 434    592    642    802    865 889
a1    a2    b1    b2    MAM    TM    931

Fig. 1D

Fig. 1E

Fig. 1G

Fig. 1F

Fig. 1H

Fig. 2A

Fig. 2B

Fig. 2C

Fig. 2D

## Fig. 3A

HUVEC

ARPE-19

- CD46
- NRP2
- THBD
- NRP2 + THBD
- α-NRP2 + THBD
- α-NRP2

## Fig. 3B

- WT
- WT + THBD
- NRP2 KO
- NRP2 KO + CD46
- NRP2 KO + THBD
- NRP2 KO + THBD + 8I21

## Fig. 3C

Overlay Pentamer-THBD dimer
with Pentamer-NRP2 dimer (shown NRP2 only)

NRP2
(a2, b1, b2)

NRP2-a1

THBD

Fig. 3D

NRP2 vs. THBD competition

Fig. 3E

NRP2-Pentamer dimer

Fig. 3F

UL128 dimer interface

Fig. 3G

Fig. 4A

Fig. 4B

Fig. 4C

Fig. 4D

Fig. 4E

Fig. 4F

Fig. 4G

Fig. 4H

Fig. 4I

## Fig. 4J

NRP2-2C12 overlay

## Fig. 4K

THBD-7I13 overlay

## Fig. 4L

NRP2 Fab competition

## Fig. 4M

THBD Fab competition

# Fig. 5

## Fig. 6A

```
mix Pentamer
with NRP2 (ectodomain)
        ↓
        SEC
        ↓
mix reconstituted
Pentamer-NRP2 complex
with excess of Fabs
13H11 and 8I21
        ↓
SEC on Superose 6
```

## Fig. 6B

Fig. 6C

scale 20 nm

## Fig. 6D

monomers          dimers

scale 20 nm

## Fig. 6F

## Fig. 6E

Fig. 6G

Fig. 6H

Fig. 6I

Fig. 7A

Fig. 7B

Fig. 7C

Fig. 7D

# Fig. 8A

# Fig. 8B

Alignment to NRP1-a2

Alignment to NRP1-b2

# Fig. 8C

```
          1        10        20        30        40        50        60        70
NRP2 001 .MDMFPLTWVFLALYFSRHQVRGQPDPPCGGRLNSKDAGYITSPGYPQDYPSHQNCEWIVYAPEPNQKIVLNFNP
NRP1 001 MERGLPLLCAVLALVLA..PAGAFRNDKCGDTIKIESPGYLTSPGYPHSYHPSEKCEWLIQAPDPYQRIMINFNP

                   80        90       100       110       120       130       140
NRP2 075 HFEIEKHDCKYDFIEIRDGDSESADLLGKHCGNIAPPTIISSGSMLYIKFTSDYARQGAGFSLRYEIFKTGSEDC
NRP1 074 HFDLEDRDCKYDYVEVFDGENENGHFRGKFCGKIAPPVVSSGPPLFIKFVSDYSTHGAGFSIRYEIFKRGP.EC

         150       160       170       180       190       200       210       220
NRP2 150 SKNFTSPNGVIESPGFPEKYPHNLDCTFTILAKPKMEIILQFLIFDLEHDPLQVGEGDCKYDWLDIWDGIPHVGP
NRP1 148 SQMHTTPSGVIKSPGFPEKYPNSLECTYIVFVPKMSEIILEFESFDLEPDSNPPGGMFCRYDRLEIWDGFPDVGP

         230       240       250       260       270       280       290
NRP2 225 LIGRYCGTKTPSELRSSTGILSLTFHTDMAVAKDGFSARYYLVHQEPLENPQCNVPLGMESGRIANEQISASSTY
NRP1 223 HIGRYCGQKTPGRIRSSSGILSMVFYTDSAIAKEGFSANYSVLQSSVSEDFKCMEALGMESGRIHSDQITASSQY

         300       310       320       330       340       350       360       370
NRP2 300 SDGRMTPQQSRLHGDDNGWTPNLDSNKEYLQVDLRFLTMLTAIATQGAISRETQNGYYVKSYKLEVSTNGEDWMV
NRP1 298 ST.NMSAERSRLNYPENGWTPGEDSYREWIQVDLGLLRFVTAVGTQGAISKETKKKYYVKTYKIDVSSNGEDWIT

         380       390       400       410       420       430       440
NRP2 375 YRHGKNHKVFQANNDATEVVLNKLHAPLLTRFVRIRPQTWHSGIALRLELFGCRVTDAPCSNMLGMLSGLIADSQ
NRP1 372 IKEQNKPVLFQGNTHPTDVVVAVFPKPLITRFVRIKPATWETGISMRFEVYGCKITDYPCSGMLGMVSGLISDSQ

         450       460       470       480       490       500       510       520
NRP2 450 ISASSTQEYLWSPSAARLVSSRSGWFPRIPQAQPGEEWLQVDLGTPKTVKGVIIQGARGGDSITAVEARAFVRKF
NRP1 447 LTSSNQGDRNWMPENIRLVTSRSGWALPPAPHSYIMEWLQIDLGEEKIVRGIIIQGGKHR......ENKVFMRKF

         530       540       550       560       570       580       590
NRP2 525 KVSYSLNGKDWEYIQDPRTQQPKLPEGNMHYDTPDIRRFDPIPAQTVRVYPERWSPAGIGMRLEVLGCDWTDSKP    SEQ ID NO: 7
NRP1 516 KIGYSNNGSDWKMIMDDSKRKAKSPEGNNNYDTPELRTFPALSTRFIRIYPERATHGGLGLRMELLGCEVEAPTA    SEQ ID NO: 6
```

## Fig. 8D

NRP2-b1 bound to VEGFC peptide

NRP1-b1 bound to SARS-CoV-2 S1 peptide

b1

NRP2

a2

b2

Pentamer

# Fig. 9A

Overlay gH-gL
Pentamer vs. Trimer

Pentamer

Trimer

# Fig. 9B

Overlay Pentamer-NRP2 (cryo-EM)
vs. Pentamer (Xtal)

Pentamer
(cryo-EM,
NRP2 not shown)

Pentamer
(Xtal PDB: 5VOB)

# Fig. 9C

Overlay Pentamer
Pentamer-2C12-7I13-13H11
vs. Pentamer-NRP2-8I21-13H11

# Fig. 9D

Overlay Pentamer
Pentamer-2C12-7I13-13H11
vs. Pentamer-THBD-13H11-MSL-109

THBD-bound Pentamer 1 THBD-bound-Pentamer 2

## Fig. 10A

## Fig. 10B

scale 20 nm

Fig. 10C

Fig. 10D

Fig. 10E

Fig. 10F

Fig. 11A

C-terminal region of THBD
contacts gL and UL130 of Pentamer 1

THBD
TME-1

THBD
linker

UL131A UL128 THBD UL131A

UL130 UL130

# Fig. 11B

## Fig. 12A

## Fig. 12B

# Fig. 12C

## Fig. 13A

Co-expression of
**gH**-Myc-Avi-His; **gL**; **UL128**
**UL130**-STREP; **UL131A**
in Expi293 cells

↓

HIS affinity purification

↓

STREP affinity purification

↓

SEC

↓

mix purified Pentamer with excess of
Fabs 13H11, 2C12 and 7I13

↓

SEC on Superose 6

## Fig. 13B

## Fig. 13C

scale 10 nm

## Fig. 13D

scale 10 nm

## Fig. 13E

# Fig. 13F

## Fig. 13G

## Fig. 13H

## Fig. 14A

UL130-UL131A-8I21
(cryo-EM Pentamer-NRP2-8I21-13H11)
UL130-UL131A-8I21
(Xtal PDB: 5VOB)

## Fig. 14B

Overlay of NRP2 with 2C12 + 7I13 + 8I21

2C12

NRP2
a2b1b2

7I13

8I21

NRP2 a1

## Fig. 14C

Overlay of THBD with 2C12 + 7I13 + 8I21

2C12

7I13

8I21

THBD

Fig. 15A

Fig. 15B

Fig. 15C

Fig. 15D
Overlay Pentamer-THBD vs. B2M

Overlay Pentamer-B2M with MHC class I

Fig. 15E

## Fig. 16A

## Fig. 16B

Coverage for human B2M

Coverage: 30 / 119 (25%)

## Fig. 16C

Fig. 16D

Fig. 16E

## Fig. 16F

## Fig. 16G

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63193529
- US 63345811
- US 6248516 B1
- WO 0000823 A
- WO 0039585 A
- US 2020025471 W
- US 2021060887 W
- WO 2020205626 A

### Non-patent literature cited in the description

- **MARTINEZ-MARTIN et al.** *Cell*, 2018, vol. 174 (5), 1158-1171, 19
- **KSCHONSAK et al.** *Cell*, 2021, vol. 184, 1232-1244, 16
- **CHANDRAMOULI et al.** *Sci. Immunol*, 2017, 2
- **PLUCKTHUN**. The Pharmacology of Monoclonal Antibodies. Springer-Verlag, 1994, vol. 113, 269-315
- **MALMBORG et al.** *J. Immunol. Methods*, 1995, vol. 183, 7-13
- **KOTTON et al.** *Nat. Rev. Nephrol.*, 2010, vol. 6, 711-721
- **HYDE et al.** *Reviews in Medical Virology*, 2010, vol. 20 (5), 311-326
- **CONNOLLY et al.** *Nat. Rev. Microbiol*, 2021, vol. 19 (2), 110-121
- **E et al.** *Proc. Natl. Acad. Sci.*, 2019, vol. 116 (14), 7043-7052
- **MARTINEZ-MARTIN et al.** *Cell*, 2018, vol. 174 (5), 1158-1171
- **NGUYEN ; KAMIL.** *Viruses*, 2018, vol. 10 (12), 704
- **MALITO et al.** *Curr. Opin. Virol.*, 2018, vol. 31, 43-51
- **MACAGNO et al.** *J. Virol.*, 2010, vol. 84 (2), 1005-1013
- **BIRON**. *Antiviral Research*, 2006, vol. 71 (2-3), 154-163
- **CHEN et al.** *Viruses*, 2019, vol. 12 (1), 21
- **DALY et al.** *Science*, 2020, vol. 370 (6518), 861-865
- **PARKER et al.** *J. Biol. Chem.*, 2012, vol. 287 (14), 11082-11089
- **WRAPP et al.** *bioRxiv*, 2021
- **SARTAIN et al.** *J. Immunol.*, 2016, vol. 196 (2), 832-845
- **BACHEM et al.** *J. Exp. Med.*, 2010, vol. 207 (6), 1273-1281
- **CHANDRAMOULI et al.** *Sci. Immunol.*, 2017, vol. 2 (30)
- **KSCHONSAK et al.** *Cell*, 2021, vol. 184 (5), 1232-1244, 16
- **KABANOVA et al.** *Nat. Microbiol.*, 2016, vol. 1 (8), 16082
- **LIU et al.** *Sci. Adv.*, vol. 7 (10), 2021
- **MEYERSON et al.** *Sci. Rep*, 2014, vol. 4, 7084
- **MASTRONARDE**. *J. Struct. Biol*, 2005, vol. 152 (1), 36-51
- **MASTRONARDE**. *J. Struct. Biol.*, 2005, vol. 152 (1), 36-51
- **SCHERES**. *J. Struct. Biol*, 2012, vol. 180 (3), 519-530
- **GRANT et al.** *eLife*, 2018, vol. 7, e35383
- **PUNJANI et al.** *Nat. Methods*, 2017, vol. 14 (3), 290-296
- **ZHENG et al.** *Nature Methods*, 2017, vol. 14, 331-332
- **ROHOU ; GRIGORIEFF**. *J. Struct. Biol.*, 2015, vol. 192 (2), 216-221
- **ROSENTHAL ; HENDERSON**. *J. Mol. Biol.*, 2003, vol. 333 (4), 721-745
- **AFONINE et al.** *Acta Crystallogr. Sect. D Struct. Biol.*, 2018, vol. 74 (6), 531-544
- **MUKHERJEE et al.** *Nature*, 2014, vol. 505 (7481), 103-107
- **WATERHOUSE et al.** *Nucleic Acids Res.*, 2018, vol. 46 (W1), W296-W303
- **EMSLEY et al.** *Acta Crystallogr. Sect. D Biol. Crystallogr.*, 2010, vol. 66 (4), 486-501
- **CROLL**. *Acta Crystallogr. Sect. D Struct. Biol*, 2018, vol. D74, 519-530
- **WILLIAMS et al.** *Protein Sci.*, 2018, vol. 27 (1), 293-315
- **GODDARD et al.** *Protein Sci.*, 2018, vol. 27 (1), 14-25
- **SIEVERS et al.** *Mol. Syst. Biol.,*, 2011, vol. 7 (1)
- **WATERHOUSE et al.** *Bioinformatics*, 2009, vol. 25 (9), 1189-1191
- **ROBERT ; GOUET**. *Nucleic Acids Res.*, 2014, vol. 42 (W1), W320-W324

- **KSCHONSAK et al.** *Cell*, 2021, vol. 184 (5), 1232-1244
- **CIFERRI et al.** *Proc. Natl. Acad. Sci.*, 2015, vol. 112 (6), 1767-1772
- **WRAPP et al.** *bioRxiv*, 2021, 2021.03.25.436804
- **APPLETON et al.** *EMBO J.*, 2007, vol. 26, 4902-4912
- **DALY et al.** *Science*, 2020, vol. 370, 861-865
- **PARKER et al.** *J. Biol. Chem.*, 2012, vol. 287, 11082-11089
- **APPLETON et al.** *EMBO J.*, 2007, vol. 26 (23), 4902-4912
- **CIFERRI et al.** *PLoS Pathog*, 2015, vol. 11 (10)
- **E et al.** *Proc. Natl. Acad. Sci. U.S.A.*, 2019, vol. 116, 7043-7052
- **GRUNDY et al.** *J. Gen. Virol.*, 1987, vol. 68 (3), 793-803
- **SMITH et al.** *Immunity*, 1996, vol. 4, 215-228
- **LI et al.** *Chin. Med. J. (Engl.)*, 2016, vol. 129, 448-455